# EUROPEAN PATENT SPECIFICATION

(11) **EP 2 691 086 B1**
(45) Date of publication and mention of the grant of the patent: **27.04.2016**
(21) Application number: 12727411.6
(22) Date of filing: 28.03.2012
(51) Int. Cl.: A61K 31/015, A61K 31/201, A61K 31/202, A61K 31/203, A61K 31/355, A61K 9/08, A61P 25/16, A61P 25/28, A61P 43/00

(54) **COMPOSITIONS FOR THE TREATMENT OF NEUROLOGIC DISORDERS**
ZUSAMMENSETZUNGEN ZUR BEHANDLUNG NEUROLOGISCHER ERKRANKUNGEN
COMPOSITIONS POUR LE TRAITEMENT DE TROUBLES NEUROLOGIQUES

(30) Priority: 29.03.2011 US 201161469081 P
(43) Date of publication of application: 05.02.2014
(73) Proprietor: Palupa Medical Ltd., 2064 Strovolos Nicosla (CY)
(72) Inventor: PANTZARIS, Marios, 2045 Strovolos Nicosia (CY); PATRIKIOS, Ioannis, Nicosia 2115 (CY); LUOKAIDIS, Georgios, Nicosia (CY)
(74) Representative: Gosmann, Martin
(86) International application number: PCT/IB2012/000824
(87) International publication number: WO 2012/131493

(56) References cited:
- EP-A1- 0 234 733
- EP-A1- 0 440 341
- EP-A1- 0 520 624
- WO-A1-96/31457

## Description

The invention relates to novel formulations to treat neurologic disorders, namely neurodegenerative diseases, autoimmune diseases and multiple sclerosis.

### BACKGROUND OF THE INVENTION

Neurologic disease is a dysfunction of the central or peripheral nervous system. It can take many forms such as degeneration of nerve cells, autoimmune disease and multiple sclerosis. Autoimmune disease is caused by antibodies or activated lymphocytes (T-cells) that attack molecules, cells or tissues of the same mammal producing them. Activated T-cells from the peripheral blood migrate into the central nervous system (CNS) and subsequently activate macrophages within the brain parenchyma at perivenular areas farming with inflammatory process to so-called multiple sclerosis (MS) plaques (lesions). B cells reflect the abnormal T-cell immunity but also have direct effects on immune regulation and brain destruction. B-cells secrete Interleukin-6 (IL-6), Interleukin-10 (IL-10), tumor necrosis factor (TNF-a) and chemokines, B-cells in MS express high levels of costimulatory molecules (CD80). As a result they are potent antigen presenting cells (APC) because they are exquisitely focused against specific antigens. New insights suggest oligodendrocyte apoptosis (degeneration) to be a primary event accompanied by microglia activation. The important pathological mechanisms involved in MS include immune mediated inflammation, oxidative stress and excitotoxicity. These mechanisms may all contribute to oligodendrocyte and neuronal damage and even cell death, hence promoting disease progression.

Multiple Sclerosis (MS) is a chronic demyelinating and degenerative disease of the CNS that attacks relatively young patients at the age of 20 to 40. About 85% of all MS cases start, with the relapsing-remitting type of the disease. Oligodendrocytes, the myelin-forming cells of the CNS, are target cells in the pathogenesis of MS. At present, the exact etiology of MS is unknown, but T-cells and macrophages are thought to be involved in demyelination through various mechanisms. For most people with MS. the disease slowly progresses with a series of unpredictable relapses (attacks of neurological symptoms). But for some, the progression
of the disease is rapid. Relapses often lead to increasing and severe disabilities such as walking impairment, muscle weakness, speech or vision impairments and many others. More than 50% of the relapsing MS patients will eventually develop severe handicaps 10 to 15 years after the onset of the disease. At present, no pharmaceutical or other therapy exists that can confer prolonged remission of MS. Current therapeutic agents (Interferons, glateramer acetate, fingolimod and monoclonal antibodies) are only partially effective. Long-term beneficial effects of existing treatments are uncertain and often detrimental side effects have been reported. For example, deaths have been associated with monoclonal antibodies such as Tysabri®. Therefore, there is a distinct need for safe and effective approaches to treating MS and other neurodegenerative diseases.

The European patent application EP 0 234 733 suggests the treatment of presenile or sensile dementia and in particular to the treatment of Alzheimer's disease. It is proposed that such conditions may be combatted or their effects alleviated by the administration of a physiologically acceptable lithium compound and/or of an essential fatty acid or a physiologically acceptable salt thereof and there are described herein methods of treatment of such conditions. The European patent application EP 0 440 341 suggests a pharmaceutical composition of GLA or DGLA and bioavailable selenium, optionally also with an 18:4 or higher n-3 EFA and/or bioavailable zinc.

### SUMMARY OF THE INVENTION

In one embodiment, the present invention relates to the use of the high dose of specific polyunsaturated fatty acids, i.e. omega-3 (eicosapentaenoic acid (EPA) and docosahexaenoic acid (DHA)), and omega-6 (linoleic acid (LA), gamma linolenic acid (GLA)), one or more other omega-3 PUFAS, and one or more monounsaturated fatty acid (MUFA) in a certain ratio resulting in normalization of essential fatty acids content in cell membranes. More particularly, the present invention relates to a combination of EPA, DHA, LA and GLA. In addition, the composition may further comprise Vitamin E, gamma-tocopherol and/or Vitamin A.

The present invention is suitable for treating human subjects who have neurodegenerative diseases, autoimmune disease and MS employing the foregoing formulations. In one embodiment, the invention is a liquid oral composition selected from the group consisting of a pharmaceutical, nutritional, medical food, functional food, clinical nutrition, medical nutrition or dietetic preparation, comprising:
(a) a long chain polyunsaturated fatty acid (PUFA) fraction, comprising eicosapentaenoic acid (EPA), docosahexaenoic acid (DHA). linoleic acid (LA) and gamma linolenic acid (GLA);
(b) one or more other omega-3 PUFAs (as defined below); and
(c) one or more monounsaturated fatty acid (MUFA); and
wherein the EPA is present in an amount of 500 mg to 5000 mg, and/or wherein the DHA is present in an amount of 1000 mg to 12000 mg.
The composition may further comprise a saturated fatty acid (SFA) and a vitamin selected from the group consisting of Vitamin A, Vitamin E and gamma-tocopherol. The LA may be present in an amount of 1000 mg to 10600 mg. The GLA may be present in an amount of 1000 mg to 16000 mg. In an embodiment according to the invention, the composition comprises 1,650 mg EPA, 4650 mg DHA, 3850 mg LA, 5850 mg GLA, 760 mg gamma-tocopherol, 22 mg Vitamin E, and 0.6 mg beta-carotene (Vitamin A).

In yet another embodiment, EPA and DHA and the other omega-3 fatty acids are administered in a triglyceride structural form to enhance absorption by the small intestine. For example, monounsaturated fatty acids are employed in combination with specific polyunsaturated fatty acids (PUFAs) and gamma-tocopherol to enhance remyelination.

Other objects, features and advantages will be set forth in the Detailed Description that follows, and in part will be apparent from the description or may be learned by practice of the embodiments disclosed herein. These objects and advantages will be realized and attained by the processes and compositions particularly printed out in the written description and claims hereof.

### BRIEF DESCRIPTION OF FIGURES

Figure 1 is a graph of the total study population conventional treatment vs. no treatment on entry baseline.
Figure 2 is a graph of the all time on study population conventional treatment vs. no treatment on entry baseline.
Figure 3 is a graph of intention to treat (ITT) population conventional treatment vs. no treatment at the end of the study.
Figure 4 is a graph of 24 month pre-entry relapses vs. 24 months post entry relapses of all-time on-study population where the numbers 22, 27, 16 and 20 denote the number of relapses of the respective group during the two years before baseline. The numbers 17, 8, 13 and 25 denote the number of relapses of the respective group during the two years after entry baseline (on treatment).
Figure 5 is a graph of number of relapses of Group B vs. placebo during the time periods of 0-12 months and 12-24 months on treatment; with 4 reported relapses in each time period within Group B but with 10 and 15 reported relapses for each time period respectively, within the placebo group.
Figure 6 is a graph of Group C showing the dispersion and frequency of the relapses during on treatment period (relapses/month).
Figure 7 is a graph of Group B with the number of relapses at every six- month period from entry baseline until study completion, against the 27 relapses that were reported for the two years pre entry period.
Figure 8 is a graph of Group A showing the dispersion and frequency of the relapses during on treatment period (relapses/month).
Figure 9 is a graph of Group B showing the dispersion and frequency of the relapses during on treatment period (relapses/month).
Figure 10 is a graph of treatment period relapses per six months per group. The first column of each set of columns per group denotes the number of relapses during the 0 to 6 month period on treatment; the second column of each set of columns per group denotes the number of relapses during the 7 to 12 month period on treatment; the third column of each set of columns per group denotes the number of relapses during the 13 to 18 month period on treatment and the forth column of each set of columns per group denotes the number of relapses during the 19 to 24 month period.
Figure 11 is a graph of annual relapse rate (ARR) x 10 at entry baseline (2 years pre entry period ARR) vs.ARR of every six months period on treatment for all-time on-study population. The first column of each set of columns represents the ARR of Group A; the second column of each set of columns represents the ARR of Group B; the third column of each set of columns represents the ARR of Group C and the forth column of each set of columns represents the ARR of Group D (the placebo).
Figure 12 is a graph of ARR x 10 of Group B vs. placebo on different time windows for all-time on-study population. The first column of each set of columns represents the Group B.
Figure 13 is a graph for Disability Progression (Mean EDSS Per Month) of all-time on-study population per treatment arm. Taking the Disability Progression axis, the very top line represents the Group A (begins at 2.65 and ends up at 3.3 mean EDSS), then is the line for Group B (begins at 2.4 and ends up at 2.7 mean EDSS), then is the line for Group D (placebo) (begins at 2.16 and ends up at 3.33 mean EDSS) and the very bottom line represents the Group C that begins at 2.11 and ends up at 2.72 mea EDSS.
Figure 14 is a Kaplan Meier graph for Sustained Progression of disability of all-time on-study population. Starting from the very top line that represents Placebo going down is then the line that represents Group A, then the line for Group C and finally the very bottom line that represents the Group B with only 10% cumulative progression of disability.
Figure 15 is a Kaplan Meier graph of Cumulative Percent EDSS Progression vs. Time of all-time on-study population. The very top line represents Group D (Placebo), then the line for Group C, then the line for Group A and the very bottom line that represents Group B.
Figure 16 is a graph of Group D (Placebo) showing the dispersion and frequency of the relapses during on treatment period (relapses/month).

### DETAILED DESCRIPTION

While the present invention is capable of being embodied in various forms, the description below of several embodiments is made with the understanding that the present disclosure is to be considered as an exemplification of the invention. Headings are provided for convenience only. Embodiments illustrated under any heading may be combined with embodiments illustrated under any other heading.

### Definitions

The term "interfering" includes either activation, inhibition, regulation, up or down-regulation of any involved pathophysiological mechanism and/or metabolic pathway in inflammation process (demyelination), remyelination, neumprotection, apoptosis, excitotoxicity, oxidative stress, gene activation, membrane receptor ligand binding, for MS and other degenerative diseases.

The term "sharing common pathophysiological mechanisms and/or metabolic pathways" refers to demyelinating, degenerative, autoimmune, cardiovascular, neurological, metabolic and genetic diseases or disorders.

The terms "polyunsaturated fatty acids" or "PUFA" or "LCPUFA" as used herein, unless otherwise specified, refer to any long chain polyunsaturated fatty acid or source thereof, having at least 18 carbon atoms per chain fatty acids having two or more carbon-carbon double bonds.

The terms "monounsaturated fatty acids" or "MUFA" or "LCMUFA" as used herein, unless otherwise specified, refer to any long chain monounsaturated fatty acid or source thereof, having at least 18 carbon atoms per chain fatty acids having one carbon-carbon double bond.

The terms "other omega-3 fatty acids," or "other omega-3 fatty acids," "other PUFA," or "other LCPUFA" as used herein, unless otherwise specified, refer to any polyunsaturated fatty acid or source thereof, having at least 18 carbon atoms per chain fatty acids having two or more carbon-carbon double bonds, with the first unsaturated double bond between the third and fourth carbon atom counting from the end methyl group of the fatty acid chain, excluding EPA and DHA.

The terms "omega-3 fatty acids," or "n-3," and "ω-3" as used herein, unless otherwise specified, refer to any polyunsaturated fatty acid or source thereof, having at least 18 carbon atoms per chain fatty acids having two or more carbon-carbon double bonds, with the first unsaturated double bond between the third and fourth carbon atom counting from the end methyl group of the fatty acid chain.

The terms "omega-6 fatty acids" or "n-6," and "ω-6" as used herein, unless otherwise specified, refer to any polyunsaturated fatty acid or source thereof, having at least 18 carbon atoms per chain fatty acids having two or more carbon-carbon double bonds, with the first unsaturated double bond between the sixth and sevcnth carbon atom counting from the end methyl group of the fatty acid chain.

The terms "saturated fatty acids" or "SFA" as used herein, unless otherwise specified, refer to any saturated fatty acid or source thereof, having at least 16 carbon atoms per chain fatty acids having no any carbon-carbon double bonds.

The terms "short chain fatty acids" as used herein, unless otherwise specified, refer to any saturated and/or unsaturated and/or polyunsaturated fatty acids or source thereof, having less than 14 carbon atoms per chain fatty acids having no any, one, two or more carbon-carbon double bonds.

The term "invention" or "intervention" was used herein, unless otherwise specified, refer to the formulations for the prevention and treatment of MS and/or other degenerative and/or autoimmune diseases or syndromes.

The term "treatment" covers and includes (a) preventing the disease from occurring in a subject which may be predisposed to the disease but has not yet been diagnosed as having it; (b) inhibiting the disease, i.e., arresting its development; or (c) relieving the disease, i.e., causing regression and/or elimination of the disease and/or its symptoms or conditions.

### Active Agents Employed in the Formulations

### Eicosapentaenoic Acid (EPA)

EPA is an important omega-3, polyunsaturated fatty acid of the marine food chain that serves as a precursor for the prostaglandin-3 and thromboxane-3 families. Merck Index at 3562 (13th Ed. 2001). EPA is also known as 20:5 (n-3); timnodonic acid; all-cis-eicosa-5, 8, 11, 14, 17-pentenoic acid; and 5 Z, 8 Z, 11 Z, 14 Z, 17 Z-eicosa-5, 8, 11, 14, 17-pentenoic acid. EPA exists as a colorless oil. As used in the present invention, the total daily dose of EPA ranges from about 500 to about 4000 mg. It is obtained from fish and microalgae or produced synthetically. In some embodiments, the EPA is in the form of re-esterified triglycerol (rTG) in the amount of about 10% to 30% (w/w).

### Docosahexaenoic Acid (DHA)

DHA is an omega-3 fatty acid found in marine fish oils and in many phospholipids. It exists as a clear, faintly yellow oil. Merck Index at 3432 (13th Ed. 2001). As used in the present invention, the total oral daily dose of DHA ranges from about 1000 to 15000 mg. DHA is also known as cervonic acid; all-cis-docosa-4, 7, 10, 13,16, 19-hexaenoic acid; 22:6 (n-3); or 4 Z, 7 Z, 10 Z, 13 Z, 16 Z, 19 Z docosa-4, 7, 10,13, 16, 19-hexaeoic acid. Cold-water oceanic fish oils are rich in DHA.. Most of the DHA in fish originates in photosynthetic and heterotrophic microalgae. DHA is also commercially manufactured from microalgae (*Crypthecodinium cohnii* and *Schizochytrium*)***.*** It can also be produced synthetically. In some embodiments, the DHA is in the form of rTO in the amount of about 30% to 70% (w/w).

### Linoleic Acid (LA)

LA is an omega-6 essential fatty acid, and is obtained by extraction from various vegetable oils such as safflower oil. It occurs as a colorless to light-yellow colored oil. Handbook of Pharmaceutical Excipients, at 414-415 (5th Ed. 2006). As used in the present invention, the total oral daily dose ranges from about 1000 to 12000 mg. LA is also known as cis, cis-9,12-octadecadienoic acid, it is found in the lipids of cell membranes. It is abundant in many vegetable oils, comprising over half (by weight) of poppy seed, safflower, sunflower, corn oils and borage oil. It can also be produced synthetically. In some embodiments, the esterified triglyceride content of LA is about 20% to 60% (w/w).

### Gamrna-Linolenic Acid (GLA)

GLA is an omega-6 polyunsaturated fatty acid from borage oil. It can also be found naturally in fish, animal organs such as liver, and certain plant seeds. It occurs as a liquid. As used in the present invention, the total oral daily dose ranges from about 1000 to about 18000 mg. GLA is also known as gamoleic acid: all-cis-6,9,12-oetadeeatrienoie acid. GLA is obtained from vegetable and seed oils such as evening primrose (*Oenothera biennis*) oil, blackcurrant seed oil, borage oil, and hemp seed oil. GLA is also found in considerable quantities in edible hemp seeds and from spirulina, a cyanobacterium. It can also be produced synthetically. In some embodiments, the esterified triglyceride content is about 30% to 60% (w/w).

### Other Omeea-3 PUFAs

The invention may also comprise one or more of 18:3, 18:4, 20:4, or 22:5 omega-3 PUFAs with a total oral daily dose ranging from about 100 to 2500 mg. Appropriately, in the composition according to the invention the other omega-3 PUFAs is present in an amount of 100 mg to 2500 mg. In certain embodiments according to the invention, the omega-3 PUFA is present in the composition in an amount of 300 mg to 2000 mg, or of 600 mg to 1000 mg.

### Monounsaturated Fatty Acids (MUFAs)

The invention may also comprise one or more of 18: 1, 20:1, 22: 1, or 24:1 MUFA with a total oral daily dose ranging from about 10 to 3500 mg. In certain embodiments according to the invention, the MUFA is present in the composition in an amount of 100 mg to 3500 mg, of 750 mg to 3500 mg, or of 1500 mg to 3500 mg.

### Saturated Fatty Acids (SFAs)

The invention may also comprise one or more of 16:0 or 18:0 SFA with a total oral daily dose ranging from about 50 to 2000 mg. In certain embodiments according to the invention, the SFA is present in the composition in an amount of 500 mg to 2000 mg.

### Gamma(γ)-Tocopherol

γ- tocopherol is fat soluble and is one of the naturally occurring forms of Vitamin E. It occurs as a pale yellow, viscous oil. Merck Index at 9573 (13th Ed. 2001). As used in the present invention, the total oral daily dose ranges from about 300 to about 3000 mg. Appropriately, in the composition according to the invention the gamma-tocopherol is present in an amount of 100 mg to 3000 mg.

### Vitamin E

Vitamin E, which typically refers to the alpha-tocopherol isoform, is a fat soluble vitamin, and as used in the present invention, it is orally administered in an amount of about 10 to 800 mg per day.

### Vitamin A

Vitamin A is a fat-soluble vitamin represented primarily by vitamin A₁ (retinol) with an empirical formula of C₂₀H₃₀O and whose four conjugated double bonds in the side chain are in the irons arrangement. Remington: The Science and Practice of Pharmacy at 1799 (20th Ed. 2000). It occurs as solvated crystals from polar sol vents such as methanol or ethyl formate. Merck Index at 10073 (13th Ed. 2001). Alpha-carotene (α-carotene) is a vitamin A precursor. The best sources for both the α- and β-isomers are carrots, palm oils, and green leaves of various species, α-carotene is found in the mother liquors after crystallizing β-carotene. It occurs as deep purple prisms. Merck Index at 1865 (13th Ed. 2001). As used in the present invention, the total oral daily dose ranges from 0.1 to 5 mg.

Other ingredients may include phospholipids, serine, inosidol, choline, ethanolamine, ascorbic acid, melatonin, testosterone, α-, β- and γ-tocotrienols, micronutrients, and antioxidants such as selenium, *Ginko biloba* extracts, coenzyme Q10, other PUFAs, other MUFAs, alpha-linolenic acid (LNA), Vitamin D, Vitamin C and alpha-lipoic acid.

The present disclosure also includes metabolites of the foregoing. For example, the formulations may comprise LA metabolites for omega-6 PUFA and LNA (alpha-linolenic acid). In another example, the formulations may comprise an effective amount of a metabolite of LA selected from the group consisting of GLA, DGLA (dihomo-gamma-linolenic acid), a 22:4n-6 and 22:5n-6 essential fatty acid and/or an effective amount of a metabolite of alpha-linolenic acid selected from the group consisting of 18:4n-3, 20:4n-3, 20:5n-3, 22:5n-3 and 22:6n-3 essential fatty acids.

### General Overview of Formulations and Use Thereof

The combination of the above ingredients have unexpectedly been shown to synergistically control, modulate, promote and/or trigger metabolic pathways leading to reduction of demyelination, promotion of remyelination and promotion of neuroprotection in MS by exhibiting a statistically significant positive effect on the total MS pathological symptoms such as (a) reduction of the annual relapse rate (ARR); (b) reduction of relapse frequency; (c) reduction of disability progression (reduction of the probability of Expanded Disability Status Scale (EDSS) score increase by one point); and (d) reduction of the development of new or enlarging T-2 lesions of the brain in Magnetic Resonance Imaging (MRI) scans, and without any significant side effects. One object of the present invention is to improve the physical status of the patients experiencing a neurodegenerative autoimmune disease, progressively accumulating disability and hence their quality of life.

Without being bound to theory, EPA/DHA omega-3 and omega-6 linoleic acid (LA)/gamma-linolenic acid (GLA) are believed to be implicated in and modulate almost all known pathways in the MS pathophysiology repertoire. For example, omega-3 and omega-6 PUFA can inhibit production of pro-inflammatory cytokines. T-cell proliferation can be reduced by supplementation with either omega-6 or omega-3 PUFAs. DHA can prevent dendritic cell maturation, T-cell stimulation and differentiation (involved in autoimmunity such as MS) and T-cell apoptosis. High intake of dietary DHA and EPA can reduce pro-inflammatory and atherogenic related gene expression. EPA and DHA have neuroprotective effects in the aged brain, are endogenous ligands of retinoid X receptors (RXRs) and peroxisome proliferator activated receptor (PPAR), and they can reverse age-related decreases in nuclear receptors and increase neurogenesis. In vitro, omega-3 PUFAs have been shown to prevent neuronal accumulations of Ca2+, which can trigger a destructive cellular cascade of events that leads to neuronal damage and death. DHA is neuroprotective against excitotoxicity, inflammation and oxidative stress that are major part of the pathogenic mechanisms. Differentiation of progenitors into mature myelin-forming oligodendrocytes is accompanied by extensive formation of new oligodendrocyte cell membranes to re-insulate demyelinated axons and PUFA may support this process. Without being bound to a theory, EPA/DHA/LA/GLA formulation is able to control and/or even halt an event so called endoplasmic reticulum "stress" (ER "stress"), probably responsible and involved in the neuronal and oligodendrocyte apoptosis and neurodegeneration.

Vitamin E (considered as alpha-tocopherol) and gamma-tocopherol are both efficiently implicated in radical scavenging with gamma-tocopherol to be highly effective in trapping nitrogen oxide radicals. Both Vitamin E and gamma-tocopherol also exert non-antioxidant properties, including modulation of cell signaling, regulation of gene transcription (i.e., genes involved in the modulation of extracellular proteins and genes connected to adhesion and inflammation), modulation of immune function and induction of apoptosis.

The preparations according to the invention can be used in the treatment and/or prevention specifically of MS, but it is also possible to be used for other neurodegenerative and/or autoimmune diseases and syndromes. It may also be beneficial for spinal cord injury recovery.

Many degenerative, autoimmune syndromes besides MS find their basic cause in common dysfunctional mechanisms and/or metabolic pathways that might all be the result of the same cause. In general, these are: common dysfunctional mechanisms and/or metabolic pathways dysfunction of the immune system, inflammation, demyelination, increased apoptotic condition, uncontrolled degenerative oxidative stress, inactivation or functional incapability for remyelination and neuroprotection. Accordingly, the present invention may be useful in the treatment of such diseases. Some of the highly common parameters that lead to the pathogenesis of all these diseases rely on specific pathways that all of them share. For example, phospholipids are the main components of nerve cell membranes. In nerve cells membranes, the middle carbon atom of phospholipids, known as Sn2, is usually attached to a long chain polyunsaturated fatty acid (LCPUFA) such as DHA, arachidonic acid (AA), and sometimes EPA. LCPUFA are fatty acids containing 18 to 26 carbon atoms with three or more double bonds. When nerve cells are activated, the activity of a group of enzymes known as phospholipase A2 (PLA2) is increased. PLA2 releases the LCPUFA from the Sn2 position, and one molecule of what is known as a lysophospholipid (LyPL) (a deacylated phospholipid without a fatty acid attached to the Sn2 position (or Sn-1 possition)) of glycerol backbone) is also released. Lysophospholipid can play a role in sustaining inflammation due to transcriptional activation of genes coding for adhesion molecules, cytokines, and growth factors. Both of these molecules are highly active cell signalling agents, and can change cell function in a many different ways. Additionally the LCPUFA can be converted to short-lived molecules such as prostaglandins, leukotrienes, hydroxy acids, that regulate neuronal function, cell growth and development.

For normal cell function, it is important that this activation to be temporary and should be terminated when LCPUFA and LyPL are removed. If this cannot be possible for some reason then this process results in membrane damage because the LyPL can be destructive. In addition, the free LCPUFA are easily oxidised to highly active free radicals that can result to great neuronal and cellular damage. There is an increased belief that these membrane damages are the major pathological basis for many neurodegenerative disorders, including, for example, multiple sclerosis, Alzheimer's disease, other dementia syndromes, Parkinson's disease, and Huntington's disease.

Signal transduction processes involving LCPUFA and LyPLs are terminated mostly when LCPUFA are linked to coenzyme A (CoA) by a group of enzymes known as acyl-CoA synthetases. The LCPUFA-CoA derivatives are then linked to the LyPL by a group of enzymes known as acyl CoA: lysophospholipid acyl transferases. This sequence thus removes from the nerve cell the LCPUFA and the LyPLs and the signal transduction triggered events are coming to an end, so preparing the neuron for the next stimulus.

In the neurodegenerative conditions there, appears to be an uncontrolled activation of membrane degrading enzymes like phospholipases, coupled with increased formation of free radicals associated with the oxidation of LCPUFA and the membrane damage produced by LyPL. Membrane damage associated with excess phospholipase activity, has been well described, for example, for multiple sclerosis, Alzheimer's disease and other dementias, in Parkinson's disease, in epilepsy, and Huntington's disease.

In all of these situations, therefore, there is some evidence of increased phospholipase activity and signal transduction activity which may not be terminated in a normal way. The common observation that EPA-enriched materials are beneficial in psychiatric disorders may therefore be explained in a. way since EPA is known to inhibit phospholipase A2 mostly by competitive inhibition against AA. EPA has an unusually high affinity for specific human brain enzyme than AA. This means that EPA will more readily than other LCPUFA enter the cycle, form an EPA-CoA derivative, link to LyPL and terminate the process and in return terminate the activity of free LyPL. Obviously EPA will, more effectively than other LCPUFAs, stop the activation once it has started. Because EPA will compete with AA for incorporation into the Sn2 position of phospholipids. EPA will also reduce the amount of AA incorporation into this position. EPA itself is a LCPUFA that can be converted to desirable protective compounds like prostaglandin PGI 3 and prostaglandin PGE 3 which are both anti-inflammatory molecules. The compounds derived from EPA appear to be much less potentially harmful than the equivalent compounds derived from AA. Replacement of AA by EPA is therefore likely to be of particular value in all the neurodegenerative disorders described above, where at least part of the damage is due to overactive phospholipases which release AA which can then be converted to pro-inflammatory compounds.

It has been widely suggested as we previously discussed that a wide range of neurological, (neuro)degenerative, psychological and autoimmune diseases/disorders, including Huntington's disease, Parkinson's disease, Alzheimer's disease and other dementias, result out of common pathogenic mechanisms with major ones, the oxidative damage of membranes, oxidative stress and activation of phospholipases. The differences between the diseases relate to the nature of the proteins and to the site of the neurons most affected, but the overall processes are similar. Some of the suggested potential therapeutic approaches include glutamate release inhibitors and radical scavengers. However, the prior art does not teach a formulation including strong antioxidant agents along with major membrane building blocks and related mechanism regulatory agents for simultaneous and synergistic treatment effect. The present invention can affect those common mechanisms that all of these diseases share. The present invention can simultaneously and synergistically affect and repair membranes, can inhibit phospholipases and can enhance antioxidant defenses. The present invention may be use as an adjuvant to conventional existing drugs for all these diseases and syndromes.

There is increasing evidence that some of the abnormalities which cause psychiatric and neurological disorders are not at the neurotransmitter or receptor level but are at the post-receptor signal transduction level. Considering the mechanism of action of MS conventional drugs, we can conclude that side effects like depression of patients receiving these drugs might be a result of post-receptor signal transduction. The present invention contains specific molecules (for example, EPA and DHA are active molecules with increased brain enzyme affinity, like EPA, for the human brain enzyme FACL-4, that are related to psychopathological disorders like depression) that can directly interfere with and possibly terminate the process of drug related depression and or other side effects.

The pathophysiological processes of these specific syndromes, and specifically MS, exhibit and share a common denominator. Without being bound to theory, the common denominator is believed to be LCPUFA. Specific LCPUFA are shown to be missing and the same LCPUFA are also shown, by one way or the other, to be able to dynamically interfere, positively or negatively with all involved pathways. These same LCPUFA sometimes are involved as enzyme inhibitors or activators, signal promoters, receptor ligands, gene activators, pathway intermediates, neuroprotectors, membrane building blocks, major myelin constituents, antioxidants, involved in apoptosis and excitotoxicity mechanisms. Additionally, these same LCPUFA that are key membrane lipid components are found in extremely low quantities compared to physiological membranes content in these patients. Accordingly, the present invention addresses can synergistically and simultaneously interfere with and effectuate treatment.

The re-esterifed form of the molecules may be used in the present invention. The term "re-esterified" is used for products made from fish body oil (FBO), in which the triglyceride (TG) content is transferred to ethyl esters and then molecularly distilled to remove the short chain and the saturated fatty acids increasing the EPA and DHA contents. The ethyl esters are then enzymatically reconverted to glycerides. Enzymatic re-esterification procedure is well known in the art. Preferably, short chain and excess amounts of SFA are removed because they may be a factor of unwanted interference of the metabolic pathways and or mechanisms that have to be normalized by the agents within the invention. In general, there is the possibility of interference in all sides of action. The availability of such short chain and excess amounts of SFA will also interfere with the aim of normalizing the already non-physiological content of cell membranes in the patients especially with MS and/or other neurodegenerative and/or autoimmune diseases or disorders. Use of these specific rTG type molecules ensures a high activity next to a relatively stable product. The enzymatic re-esterification procedure is well known in the art.

In one embodiment, the present inventors have now unexpectedly and surprisingly determined that treatment with a formulation comprising re-esterfied triglycerol (rTG) EPA, DHA, accompanied with other omega-3 fatty acids within the rTG structure, TG LA, GLA, accompanied with MUFAs and SFA within the TG structure, gamma-tocopherol, vitamin A and vitamin E, among the agents of the invention, provides statistically significant positive results in all evaluation treatment characteristics of MS.

The unexpected findings that the invention is able to maintain the patients at the relapsing remitting (RR) phase together with the first line conventional treatment (interferons, glatiramere acetate) for a much longer period than the conventional treatment alone, result in the delayed progression of the disease, where much more toxic second-line drugs are used. As a result, the present invention provides a valuable contribution to the patients' treatment and quality of life.

Thus, the present invention provides preparations useful for the prevention and/or treatment of MS, for the treatment of any neurodegenerative disease or being at risk of developing any neurodegenerative disease, any psychiatric disease or being at risk of developing any psychiatric disease, any other degenerative disease or being at risk of developing any degenerative disease, any autoimmune disease or being at risk of developing any autoimmune disease, any immune mediated inflammation or being at risk of developing any immune mediated inflammation, any inflammation or being at risk of developing any inflammation, any cardiovascular disease or being at risk of developing any cardiovascular disease, epileptogenesis and epilepsy or being at risk of developing epileptogenesis or epilepsy. In one embodiment, the inventive oral liquid formulation comprises the following fractions:
- Fraction (a) comprising omega-3 long chain polyunsaturated fatty acids (LCPUFA);
- Fraction (b) comprising of omega-6 LCPUFA, which fraction contains at 3 to 4 (or more) different MUFA molecules selected from the group of LCMUFA with no more than a 24 carbon chain and no less than a 18 carbon chain, which fraction contains at least 1 to 2 different saturated fatty acids (SFA) molecules selected from the group of long chain fatty acids with no more than a 20 carbon chain and no less than a 16 carbon chain;
- Fraction (c) comprising of gamma-tocopherol; and
- Fraction (d) comprising an antioxidant.

As further described below, the invention is a pharmaceutical, nutritional, medical food, functional food, clinical nutrition, medical nutrition or dietetic preparation. The invention can be in the form of a liquid, powder, bar, cookie, dessert, concentrate, paste, sauce, gel, emulsion, tablet, soft gel capsule, hard gelatin capsule, other type of capsule or other dosage form to provide the daily dose of the bioactive components either as a single dose or in multiple doses. The compounds may also be administered parenterally, either directly, or formulated in various oils or in emulsions or dispersions, using either intravenous,' intraperitoneal, intramuscular or subcutaneous routes. The products can be packaged by applying methods known in the art, to keep the product stable during shelf life and allow easy use or administration.

The administration of the preparations of invention results in the treatment and prevention of MS and for the treatment of any, neurodegenerative disease or being at risk of developing any neurodegenerative disease, any psychiatric disease or being at risk of developing any psychiatric disease, any other degenerative disease or being at risk of developing any degenerative disease, any autoimmune disease or being at risk of developing any autoimmune disease, any immune mediated inflammation or being at risk of developing any immune mediated inflammation, any inflammation or being at risk of developing any inflammation, any cardiovascular disease or being at risk of developing any cardiovascular disease, epileptogenesis and epilepsy or being at risk of developing epileptogenesis or epilepsy. Without being bound by theory, the invention causes the simultaneous interference of mechanisms involved in MS pathogenesis, and orchestration of related mechanisms involved in resolution, normalization, restoration, remyelination, degeneration and neuroprotection for MS. In particular, the mechanisms involved in relation to the disease pathogenesis is immune related inflammation, demyelination, oxidative stress, excitotoxicity, degeneration, remyelination and neuroprotection.

Fraction (a) comprises long chain polyunsaturated fatty acids, preferably omega-3 fatty acids.

Fraction (b) comprises long chain polyunsaturated fatty acids, for example, omega-6 fatty acids. Further fatty acids that can be present are MUFA and SFA.

The mixture of omega-3 (of EPA and DHA) and omega-6 (of LA and GLA) long chain polyunsaturated fatty acids (LCPUFA) may be included in a ratio of omega-3 LCPUFA to omega-6 LCPUFA of about 1 to 1 (w/w).

One aspect includes omega-3 LCPUFA as a mixture of the EPA and DHA omega-3 LCPUFA, together with other omega-3 LCPUFA. Another embodiment includes omega-6 (LA and GLA) with a mixture of MUFA and SFA.

Advantageous treatment results are obtained when DHA and EPA are included in a ratio of DHA to EPA of about 1 to 1, 1 to 2, 1 to 3, 1 to 4, 1 to 5, 2 to 1,3 to 1, 4 to 1, or 5 to 1 (w/w). Further, other omega-3 LCPUFAs that can be present are the 18:3 (alpha-linolenic acid), 18:4 (stearidonic acid), 20:4 (eicosatetraenoic acid), 22:5 (docosapentaenoic acid) and other omega-3 LCPUFA molecules.

In one embodiment, omega-6 LCPUFAs are linoleic acid (LA) and gamma-linolenic acid (GLA). Advantageous results are obtained when LA and GLA are included in a ratio of LA to GLA of about 3 to 1, 2 to 1, 1 to 1 (w/w). Further fatty acids that can be present are the MUFAs 18:1 (oleic acid), 20:1 (eicosenoic acid), 22:1 (docosenoic acid), and 24:1 (tetracosenic acid), and SFAs 16:0 (palmitic acid), and 18:0 (stearic acid).

In one embodiment of the invention, LA and GLA in the fatty acid composition are present in the composition in an LA to GLA ratio from about 1 to 1 up to about 5 to 1 (w/w). In another embodiment the LA to GLA ratio in the fatty acid composition is from about 1 to 1 up to 3 to 1 (w/w).

In another specific embodiment of the invention, omega-3 LCPUFA, the DHA, EPA and the other omega-3 fatty acids, is comprised of a combination of EPA, DHA and the other omega-3 fatty acids in re-esterified triglyceride (minimum value 60%), diglyceride (about 33%), monoglyceride (about 2%) structural form mixture and about 2% ethyl ester structural form. All glyceride fractions contain EPA, DHA and other omega-3 fatty acids. Advantageous results are obtained when invention is comprised of EPA, DHA and other omega-3 fatty acids in at least 60% re-esterified triglycerol form.

Advantageous results are obtained when omega-3 LCPUFA are in re-esterified triglycerol (rTG) form with no less than 80% rTG content to be DHA and EPA preferably in the range of at least about 80-96%, as a result of LCPUFA triglycerides re-esterification of fish body oils (FBO). Beneficial results are obtained when total other omega-3 as rTG content is no less than about 4%-20%.

In one embodiment, EPA rTG content value is about 8% (about 72 mg/g of fraction (a)) to 26% (234 mg/g of fraction (a)), or EPA rTG content value is about 17% (153 mg/g of fraction (a)). Preferable DHA rTG content value of about 24% (216 mg/g of fraction (a)) to 78% (702 mg/g of fraction (a)), more preferably about 50% (459 mg/g of fraction (a)). Further presence of other LCPUFA is present in this embodiment and best results are obtained when no less than 2, or 3 or 4 LCPUFA out of the 18:3 (alpha-linolenic acid), 18:4 (stearidonic acid), 20:4 (eicosatetraenoic acid), 22:5 (docosapentaenoic acid) omega-3 LCPUFA molecules occupy the free Sn position(s) on the re-esterified triglycerol along with EPA and DHA.

Preferable total (EPA+DHA+ other Omega-3) omega-3 LCPUFA as rTG value of about 60-85% (preferable 66%, minimum 600 mg/g of fraction (a)). Advantageous results are obtained when the enzymatic re-esterification process is the method of re-esterification with EPA and DHA randomly positioned on the glycerol, meaning approximately 33% of EPA and DHA at the Sn1 position, 33% of EPA and DHA at Sn2 and 33% of EPA and DHA at Sn3 position.

In a specific embodiment of the invention omega-3 LCPUFA can be natural or chemically produced in the form of ethyl esters, free fatty acids, mono-, di-, or tri- glycerides, phospholipids, amides or fatty acid salts as free molecules individually added or supplied through the addition of specific marine or chemically composed oil with molecular content components within the ranges and molecular structure as denoted.

Beneficial results are obtained when omega-6 LCPUFA are in esterified triglycerol (TO) form with no less than 30-70% TG content to be LA and GLA or about 55-65%. About 20-60% of the TG should have LA at the Sn-1, or Sn-3 position, preferably at least 35%. About 20-60% of the TG should have GLA at the Sn-2 position, preferably at least 40%. Beneficial results are obtained when the total LA TG content is 20-45% (200 mg/g to 450 mg/g of fraction (b)) preferably at least 35-42% (350 mg/g to 420 mg/g of fraction (b)) and more preferably 380 mg/g of fraction (b), the total GLA TG content is 15-40% (150 mg/g to 400 mg/g of fraction (b)) preferably at least 15-22% (150 mg/g to 220 mg/g of fraction (b)) and more preferably 180 mg/g. Further presence of MUFA may be used and advantageous results are obtained when no less than 2, or 3 or 4 different MUFA molecules are selected from the group of 18:1 (oleic acid), 20:1 (eicosenoic acid), 22:1 (docosenoic acid), 24:1 (tetracosenic acid) MUFA molecules and both 16:0 (palmitic acid), 18:0 (stearic acid) SFA molecules, to occupy the free Sn position(s) on the TG.

In other embodiments, beneficial results are obtained when 10-30% of TG content is MUFA where oleic acid is preferably at least 14-20%. Excellent results are obtained when other MUFA (eicosenoic acid, docosenoic acid, tetracosenic acid) content is 3-15% and most preferably 5-10%; and SFA content, 4-16% is palmitic acid and 1-10% is stearic acid and most preferably 8-12% palmitic acid and 2-5% stearic acid.

The daily oral dose of the total of EPA+DHA+LA+GLA in one embodiment is 3000 mg to 22000 mg. In another embodiment, the dose is 12000 mg per day, comprising 4650 mg DHA, 1650 mg EPA, about 2000 mg GLA, and 3850 mg LA.

In another embodiment, the daily dosage of the total of 18:3, 18:4, 20:4, 22:5 other omega-3 LCPUFA is 300 mg to 2400 mg, or 600-1000 mg. However, the ratio of the total amount of 18:3, 18:4, 20:4, 22:5 LCPUFA to the total amount of EPA+DHA+LA+GLA should be larger than 0.04 wt/wt, but no larger than 0.10 wt/wt. Beneficial results were obtained with 0.06 wt/wt.

The daily dosage of the total of 18:1,20:1, 22:1, 24:1 MUFA molecules is 1500 mg to 3500 mg or 2500 mg, with 18:1 (oleic acid) 1300 mg, and the rest of MUFA (20:1,22:1,24:1) 500 mg.

The daily dosage of the total of 16:0, 18:0 SFA molecules is 500 mg to 2000 mg, or 1300 mg, with 16:0 650 mg to 1000 mg and 18:0 150 mg to 450 mg. However, the ratio of the total amount of MUFA to SFA should be larger than 1.0 wt/wt.

The ratio of 18:1, 20:1, 22:1, 24:1 MUFA to the total amount of EPA+DHA+LA+GLA should not be larger than 0.20 wt/wt, and the ratio of 16:0, 18:0 SFA to the total EPA+DHA+LA+GLA should not be larger than 0.10 wt/wt.

Omega-6 LCPUFA, MUFA and SFA can be natural or chemically produced in the form of ethyl esters, free fatty acids, mono-, di-, or tri- glycerides, amides, phospholipids or fatty acid salts as free molecules individually added or supplied through the addition of any vegetable or chemically composed oil with molecular content components within the ranges and molecular structure as denoted.

Without being bound to theory, the function of fraction (a) and (b) is to supply the subject with a high dose of omega-3 and omega-6 (about 1 to 1 wt/wt) that is well above of the normal daily diet consumption habits, in relation to these PUFA content, of the population of all countries. One aim is to equilibrate the subjects' PUPA intake with an overall omega-3 and omega-6 fatty acids consumed daily within the ratio of about 1 to 1 wt/wt. This is to ensure normalization and adaptation of the subject according to the recommended daily ratio of omega-3 to omega-6 fatty acid, about 1 to 1 wt/wt independently of its normal daily consumption by the population through diet habits (in relation to omega-3 and/or omega-6). For example, in the industrialized countries and specifically in USA today the ratio of omega-3 to omega-6 fatty acid has reached the well above the normal ratio of 1 to 15 wt/wt. Normalization of the diet will result to the normalization of the cellular membrane content in respect to these specific LCPUFA and specifically of the cells of interest, in relation to the MS and at the same time to their interference with all the mechanisms involved for the MS treatment. The fatty acid composition of phospholipids determines biophysical (and functional) characteristics of membranes (e.g., membrane fluidity, transport, etc.), and plays an important role in overall cellular integrity, and intra- and inter-cellular communication (signaling).

Omega-3 and omega-6 LCPUFA play a fundamental synergistic role in the related mechanisms and biological pathways in relation to the MS pathophysiology: inflammation, demyelination, excitotoxicity, degeneration, apoptosis, neuroprotection and remyelination. Overall, fatty acids can affect leukocyte function by different mechanisms of action; (a) activation of intracellular signaling pathways; (b) activation of lipid-raft-associated proteins; (c) binding to toll-like receptors (TLRs); (d) regulation of gene expression; (e) activation of transcription factors; (f) induction of cell death; (g) production of eicosanoids; (h) production of reactive oxygen species (ROS); and (i) production of reactive nitrogen species (RNS). PUFAs may also interfere with the production of certain matrix metalloproteinases (MMPs) that can be the cause of disruption of the blood brain barrier (BBB) that normally protects brain neurons.

Omega-3 fatty acids EPA and DHA that have neuroprotective effects are endogenous ligands of retinol X receptor (RXR) and peroxisome activated receptors (PPAR), will activate RXR-gamma that is a positive regulator of endogenous oligodendrocyte precursor cell differentiation and remyelination. DHA supplementation will also increase possible receptor expression as a result of any additional mechanisms that might underlie neuroprotective and remyelination effects of omega-3 fatty acids and/or EPA/DHA positive effect on neuroprotection and/or remyelination mechanisms and/or metabolic pathways.

Omega-3 LCPUFA will be involved in neuroprotection but also in the mechanisms of controlling the oxidative stress, the inflammatory reaction, the neuronal and oligodendrocyte survival and axonal damage recovery. Lipid peroxidation, protein oxidation, and RNA/DNA oxidation will all significantly be reduced by the DHA administration. In such case, increased amounts of DHA and/or EPA requires the presence of antioxidant molecules, like Vitamin A, Vitamin E and gamma-tocopherol to prevent peroxidation of excess membranes' PUFA. Induction of cyclooxygenase COX-2 in the presence of omega-3 LCPUFA results in the inhibition of the production of inflammatory cytokines, chemokines and adhesion molecules. As a result, macrophage recruitment will be reduced and neuronal and oligodendrocyte survival will substantially increase.

LCPUFA will also induce and accelerate myelinogenesis and this is an extra reasoning for the LCPUFA use in the therapeutical approaches of demyelinating diseases. LCPUFAs will alter the function of oligodendrocytes by affecting their membrane composition and membrane polarisation favoring protein phosphorylation of myelin basic protein by omega-6 PUFA in oligodendrocytes, an important event in myelination. LCPUFA will upregulate production of the mRNA levels of specific oligodendrocyte myelin proteins for remyelination. Levels of proteolipid protein, myelin basic protein, and myelin oligodendrocyte protein mRNAs will be increased in nearly all brain regions. LCPUFA will additionally result in increased levels of the myelination protein CNPase.

Increased amount of DHA is required to normalize the pathogenic neuron cells that are normally mostly composed by DHA LCPUFA. As a result a major quantity of the supplied DHA will be used for this action target (high dietary alpha-linolenic acid (LNA) increases the LNA, but not the DHA contents in brains of suckling rats. Thus, when increased DHA in the brain is required, DHA itself, and not LNA, should be administered. This is the reason of not using LNA as major formula invention component). In addition, some of the supplement DHA can be the source of EPA as well, through retro-conversion mechanism and this is another reason for increased use of DHA in relation to EPA.

Without being bound to theory, the function and role of the further added LCPUFA, MUFA and SFA, in addition to the EPA, DHA, LA and GLA of fractions (a) and (b) is to provide a direct source of neuronal cell phospholipids, for myelin reconstruction, remyelination and neuroprotection as they are the building blocks of any new physiological myelin and other cell membranes as well. A fraction of these molecules will also be used in part as energy source needed for normal cell formation and normal function. Cell membrane bilayers cannot be exclusively composed and formed by PUFA because these cell membranes are going to be characterized with abnormal high fluidity and the cells will burst, as a result of the saturations of the PUFA chains and their structural conformation within the bilayer. Limited quantities of SFA along with MUFA and PUFA ratio will equilibrate the physiological composition content of the newly formed biomembranes along with the available cholesterol and structural proteins. The usual SFA found in normal biomembranes as part of phospholipids are: stearic acid and palmitic acid (as one out of the two fatty acids is found on the phospholipids backbone). The most usual MUFA found in normal biomembranes again as a phospholipid part is the oleic acid. The formation of new myelin requires to be consisted of different LCPUFA, PUFA, MUFA and in less amounts of some SFA in order to have physiological fluidity, mobility and integrity in order to exhibit physiological and normal functions. The availability of these molecules will also support the prevention abilities of the invention formula by normalizing their content in the existing neuron cells and in all other cellular membranes. In a way they can be considered as necessary agents to help and function as neuroprotectors. These additional molecules will be part of phospholipids as well as the LCPUFAs DHA, EPA, LA, and GLA. In pathological conditions where the cause pathogenic mechanism is partially due to the non physiological content of the cell membrane components, the expectation of reversing these conditions without treating the cause is unreal. In such conditions the physiological cell membrane lipid-fatty acid components have to be available for use and for the reversal of the pathogenic mechanisms. Some of these molecules needed for the normalization of membranes' lipid-fatty acid content can be produced through different metabolic pathways, but still the appropriate raw material has to be provided and be present at the side and no other condition can ensure this but the normalization of the diet consumed. In addition, their availability when needed cannot be ensured, especially for the reformation of a physiologically functioning structure such as myelin within an organism that is experiencing problems as a result of related molecular components deficiency. Specific enzymes of lipid metabolism might also be deficient within these MS patients and as a result the needed molecules are required to be consumed through diet instead of been formed as required by the organism. After all limited and balanced quantity of SFA of specific carbon chain length is also required for the formation of cell membranes with normal fluidity, mobility, integrity and physiological functions.

As described above, fraction (c) comprises gamma-tocopherol. The daily dose of gamma-tocopherol may be about 100 mg, about 200 mg, about 500mg, about 1000mg, or about 1500mg. Beneficial results are obtained when about 760 mg of natural gamma-tocopherol isoform were used in the inventive formulations. In an embodiment, such a composition according to the invention comprises 1650 mg EPA, 4650 mg DHA, 3850 mg LA, 5850 mg GLA, 760 mg of gamma-tocopherol, and 22 mg Vitamin E. Gamma-tocopherol can also be supplied as chemically synthesized in the form of free gamma-tocopherol, salt, or esterified or as natural gamma-tocopherol in esterified form or as a salt.

Fraction (d) provides anti-oxidant properties and comprises the antioxidants vitamin A preferentially in the form of beta-carotene and vitamin E (alpha-tocopherol isoform). The daily dosage of vitamin A is between about 0.1 mg to 5 mg, about 0.6 mg to 1.5 mg, or about 0.6 mg. The daily dose of vitamin E is between about 15 mg to 50 mg, or about 22 mg. Any other carotenoid or lipoic acid can be used. Vitamin C and selenium salts can also be included.

The invention can contain any further single or different combined agents comprising any naturally and/or chemically, and/or molecularly and/or in any other way prepared and/or synthesized interferons and/or glatiramere acetate and/or mitoxantrone, and/or natalizumab and/or daclizumab, and/or alemtuzumab and/or rituximab, and/or any other monoclonal antibody and/or cladribine, and/or fingolimod and/or BG-12 and/or dimethyl fumarate and/or teriflunomide and/or anti-lingo and/or neurotrophins and/or neurosteroid dehydroepiandrosterone (DHEA) and/or vitamin D and/or antibiotic and/or immunosuppressant agent and/or any other chemically, molecularly and/or in any other way prepared and/or synthesized substance for the treatment of MS and/or any other degenerative, autoimmune diseases/syndromes.

The PUFA and/or MUFA and/or SFA components of the liquid composition may further comprise, in addition to the specific denoted EPA+DHA+LA+GLA LCPUFA and the 18:3+18:4+20:4+22:5 other omega-3 and the 18:1+20:1+22:1+24:1 MUFA and the 16:0+18:0 SFA components as described above, any other lipids and/or fatty acids suitable for use in an oral nutritional and/or pharmaceutical product. These other lipids and/or fatty acids suitable for use within the liquid composition may include the addition of other MUFA than the 18:1, 20:1, 22:1, 24:1, different other omega-3 PUFA than the 18:3, 18:4, 20:4, 22:5, different other omega-6 PUFA than the LA such as DGLA, and/or other SFA than the 18:0 and 16:0, or short chain (less than 6 carbon atoms), medium (from 6 to 16 carbon atoms) or long chain fatty acids (at least 18 carbon atoms) or be used as substitute of the denoted FAs.

### Formulation Examples 1-10

In other embodiments, compositions are prepared according to the formulation examples below.

| **Ingredient (mg)** | **1** | **2** | **3** | **4** | **5** | **6** | **7** | **8** | **9** | **10** |
|---|---|---|---|---|---|---|---|---|---|---|
| **EPA** | 800-4000 | 500-2500 | 1650 | 800-2500 | 1250-2500 | 750-2000 | 1500-2000 | 1600-1700 | 1000-2000 | 1500 1750 |
| **DHA** | 2400-12000 | 1500-7500 | 4650 | 2400-7500 | 3750-7000 | 2500-5000 | 3000-5000 | 4000-5000 | 4500-5000 | 4000-6000 |
| **LA** | 2200-10600 | 1400-6600 | 3850 | 2200-6600 | 3400-5280 | 2500-5000 | 3500-4000 | 3500-4500 | 2000-5000 | 4000-5000 |
| **GLA** | 1100-16000 | 700-3300 | 2000 | 1100-5300 | 1100-3300 | 5850 | 1700-2650 | 3300-16000 | 3000-9900 | 5100-8000 |
| **Alpha-linolenic acid** | 0-2500 | 300-2400 | 600-1000 | 300-2000 | 100-1000 | 200-900 | 300-800 | 300-600 | 200-500 | 200-750 |
| **Stearidonic acid** | 0-2500 | 300-2400 | 600-1000 | 300-2000 | 0-2000 | 0-1500 | 0-1000 | 0-750 | 0-500 | 0-300 |
| **Eicosatetraenoic acid** | 0-2500 | 300-2400 | 600-1000 | 300-2000 | 0-3000 | 0-2000 | 0-1750 | 0-1500 | 0-1000 | 0-500 |
| **Docosapentaenoic : acid** | 0-2500 | 300-2400 | 600-1000 | 300-2000 | 0-3000 | 0-2000 | 0-1750 | 0-1500 | 0-1000 | 0-500 |
| **Oleic acid** | 0-3500 | 1300-3500 | 1300 | 0-2500 | 0-2000 | 0-1750 | 0-1500 | 0-1250 | 0-1000 | 0-500 |
| **Eicosenoic acid** | 0-3500 | 250-420 | 250 | 0-2000 | 0-1500 | 0-1250 | 0-500 | 0-1000 | 0-2500 | 200-300 |
| **Docosenoic acid** | 0-3500 | 80-250 | 82 | 0-2500 | 0-2000 | 0-1500 | 0-1000 | 0-750 | 0-500 | 10-90 |
| **Tetracosenic acid** | 0-3500 | 80-160 | 82 | 0-2500 | 50-200 | 80-250 | 0-1000 | 0-750 | 0-500 | 10-90 |
| **Palmitic acid** | 0-2000 | 650-1000 | 650 | 0-2500 | 50-800 | 500-750 | 0-1000 | 0-3000 | 500-1000 | 600-700 |
| **Stearic acid** | 0-2000 | 150-450 | 160 | 100-200 | 50-200 | 0-200 | 0-1000 | 0-3000 | 100-500 | 150-750 |
| **Gamma tocopherol** | 0-3000 | 200-2000 | 760 | 500-3000 | 500-2000 | 500-1500 | 170-800 | 500-1000 | 200-1000 | 600-800 |
| **Vitamin E** | 0-50 | 15-40 | 22 | 15-500 | 20-800 | 15-200 | 20-30 | 20-50 | 20-25 | 0-500 |
| **Vitamin A** | 0-5 | 0.3-2 | 0.6 | 0.6-3 | 0.3-1.5 | 0-7 | 0.1-1 | 0.1-0.75 | 0-1 | 0.2 |

In other embodiments, compositions are prepared according to the formulation example below. Substitutes for, and metabolites of, omega-6 and omega-3 can be employed. The omega-6 metabolic pathway is set forth as follows: 18:2 LA (linoleic acid) to 18:3 GLA gamma-linolenic to 20:3 DGLA (dihomo-gamma-linolenic) to NO interested Arachidonic Acid (inflammatory). The omega-3 metabolic pathway is set forth as follows: 18:3 alpha-linolenic acid to 18:4 stearidonic acid to 20:4 eicosatetraenoic Acid to 20:5 eicosapentaenoic acid to 22:5 docosapentaenoic acid to 24:5 tetracosapentaenoic to 24:6 tetracosahexaenoic to 22:6 docosahexaenoic acid.

For example, the present invention relates to a composition for treating unsaturated fatty acid deficiencies in neurodegenerative diseases, and autoimmune diseases, and MS patients for administering to these patients:
(a) An effective amount of a metabolite of 18:2n-6 (linoleic acid (LA)) selected from, the group consisting of 18:3n-6 (gamma-linolenic acid (GLA)), and 20:3n-6 (dihomo-gamma-linolenic (DGLA));
(b) An effective amount of a metabolite of 18:3 (alpha-linolenic (ALA)) selected from the group consisting of 18:3n-3(alpha-linolenic (ALA)), 18:4n-3 (Stearidonic Acid (SA)), 20:4n-3 (Eicosatetraenoic Acid (ETA)), 20:5n-3 (Eicosapentaenoic Acid (EPA)), 22:5n-3 (Docosapentaenoic (DP A)), 24:5n-3 (tetracosapentaenoic (TPA)), 24:6n-3 (tetracosahexoenoic (THA)), and 22:6n-3 (Docosahexaenoic (DHA)) essential fatty acids;
(c) An effective amount of gamma-tocopherol; and/or
(d) An effective amount of vitamin A (alpha- or beta- carotene) and or vitamin E.

For example, SFA can be 14:0 and/or 20:0. All of the above can be in a form of phospholipid, mono, di, tri-glycerol free fatty acid, methyl or ethyl ester, or fatty acid salts naturally or chemically produced, as free molecules individually added or supplied though the addition of any vegetable or chemically composed oil with molecular content components within the ranges and molecular structure as described herein.

Omega-3 and omega-6 PUFA have an additional powerful effect on fat metabolism and they can lower insulin levels within the body by more than 50%. Since insulin inhibits the metabolism of storage fat for energy this can lead to considerable weight, loss. Insulin increases the activity of an enzyme known to promote the storage of fat. Insulin inhibits the action of hormone sensitive lipase, which is responsible for breaking down stored fat and preparing it for use as energy. Insulin also activates an enzyme, which, along with fatty acid synthesis, is responsible for converting carbohydrate into fat. High levels of insulin make it less likely that the body will use stored fat as a fuel source. The drop in insulin levels allow more fat to be used for energy.

The invention may also be useful in anti-aging, increasing libido, hair growth, premenstrual syndrome, asthma, rheumatoid arthritis, other types of arthritis, diabetes, cancer and skin diseases.

Further to the proposed agents, for example, the following can be used as part of the formula: phospholipids, phosphadityl ethanolamine, phosphadityl serine, phosphadityl inositol, phosphadityl choline, serine, inosidol, choline, ethanolamine, "other" PUFA and MUFA, alpha-linolenic, mono and/or poly hydroxyl PUFA, mono and/or poly hydroxyl MUFA, mono and/or poly hydroxyl omega-3 and/or omega-6 and/ or "other" mono and/or poly hydroxyl PUFA and MUFA and or mono and/or poly hydroxyl SFA, mono and/or di PUFA and/or MUFA and/or SFA and/or omega-3 and/or omega-6 and/or "other" PUFA and MUFA and/or SFA phospholipids and/or in any combination of those as lipid backbones, PUFA and/or MUFA and/or SFA dimmers and/or polymers, mono and /or poly hydroxyl PUFA and/or MUFA and/or SFA dimmers and/or polymers, and/or as mono, di or tri glycerols, and/or as free fatty acids, and/or as salts, and/or as methyl or ethyl esters, Vitamin D, Vitamin C, melatonin, testosterone, micronutrients and antioxidants such as selenium, *Gingko. biloba* extracts, coenzyme Q10, alpha lipoic acid, glutathione, thiol-based antioxidants, flavonoids, curcumin from *curcuma longa* (diferuloyl methane), any α-, β-, γ-, δ-tocotrienols, β**-**, 8-tocopherols, N-acetylcysteine, dihydrolipoic acid, alpha-carotene, quercetin (a flavonoid phytoestrogen), apigenin, kaempferol, naringenin, estrogen, luteolin, and cannabis, Echium oil, a natural vegetable oil rich in short-chain omega-3 polyunsaturated fatty acids *(Echium plantaginemn,* commonly known as Purple Viper's Bugloss or Paterson's Curse), or short-chain omega-3 polyunsaturated fatty acids extracts from fish oil or from any other source, or short chain, omega-6 polyunsaturated fatty acids extracts from borage oil or from botanical or any other source.

Our proposed agents and the above other agents can be used as a whole or as a part of the formula or some as substitutes in the form of liposome, micelles or as bilayer sheets.

The invention formula may advantageously in some patients be co-administered with other drugs used in neurology and psychiatry. Such drugs may include drugs of the typical neuroleptic class such as, for example, chlorpromazine, haloperidol, thioxanthene, sulpiride, pimozide; drugs of the atypical neuroleptic class including, sertindole, ziprasidone, quetiapine, zotepine and amisulpiride; drugs which have antidepressant actions including related antidepressants, noradrenaline reuptake inhibitors, serotonin reuptake inhibitors, monoamine oxidase inhibitors and drugs with atypical antidepressant actions: for example, drugs for sleep disorders, anxiety disorders, panic disorders, social phobias, personality disorders; drugs for any form of dementia, including Alzheimer's disease, vascular and multi-infarct dementias, Lewy body disease and other dementias; drugs for any form of neurological disease including Parkinson's disease, Huntington's disease and other neurodegenerative disorders.

In each of the above cases, the invention compound and the other drug may be administered separately, each in their own formulation. They may be packaged separately or be present in the same overall package. Alternatively, using techniques well known to those skilled in the art, the invention formula dosage and other drug may be formulated together, so that a daily dose of the invention formula as previously described is provided with the normal daily dose of the other drug.

The compositions described herein can be prepared in a variety of forms and contain ingredients beyond those described above.

### Pharmaceutical Excipients

Various embodiments can, if desired, include one or more pharmaceutically acceptable excipients. The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition. Excipients include, by way of illustration, diluents, disintegrants, binding agents, adhesives, wetting agents, lubricants, glidants, surface modifying agents, substances added to mask or counteract a disagreeable taste or odor, flavors; dyes, fragrances, and substances added to improve appearance of the composition. Any such excipients can be used in any dosage forms according to the present disclosure, including liquid, solid or semi-solid dosage forms.

Excipients optionally employed in various embodiments can be solids, semi-solids, liquids or combinations thereof. Compositions of the disclosure including excipients can be prepared by various pharmaceutical techniques such as admixing an excipient with a drug or therapeutic agent.

In various embodiments, compositions optionally comprise one or more pharmaceutically acceptable diluents as excipients. Suitable diluents illustratively include, for example, either individually or in combination, lactose, including anhydrous lactose and lactose monohydrate; starches, including directly compressible starch and hydrolyzed starches (e.g., Celutab™ and Emdex™); mannitol; sorbitol; xylitol; dextrose (e.g., Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of alpha and amorphous cellulose (e.g., Rexcel™) and powdered cellulose; calcium carbonate; glycine; bentonite; polyvinylpyrrolidone. Such diluents, if present, may constitute in total about 5% to about 99%. about 10% to about 85%. or about 20% to about 80%, of the total weight of the composition. In various embodiments, the diluent or diluents selected may exhibit suitable flow properties and, where tablets are desired, compressibility.

The use of extragranular microcrystalline cellulose (that is, microcrystalline cellulose added to a wet granulated composition after a drying step) can be used to alter or control hardness (for tablets) and/or disintegration time.

In various embodiments, compositions optionally comprise one or more pharmaceutically acceptable disintegrants as excipients, such as in tablet formulations. Suitable disintegrants include, for example, either individually or in combination, starches, including crosslinked polyvinylpyrrolidone (crospovidone USP NF), carboxymethyl cellulose (sodium CMC), chitin, chitosan, sodium starch glycolate (e.g., Explotab™ of Pen West) and pregelatinized corn starches (e.g., National™ 1551, National™ 1550, and Colocorn™ 1500), clays (e.g., Veegum™ HV), celluloses such as purified cellulose, microcrystalline cellulose, methylcellulose, carboxymethylcellulose and sodium carboxymethylcellulose, croscarmellose sodium (e.g., Ac-Di-Sol™ of FMC), alginates, and gums such as agar, guar, xanthan, locust bean, karaya, pectin and tragacanth gums.

Disintegrants may be added at any suitable step during the preparation of the composition, particularly prior to a granulation step or during a lubrication step prior to compression. Such disintegrants, if present, may constitute in total about 0.2% to about 30%, about 0.2% to about 10%, or about 0.2% to about 5%. of the total weight of the composition.

In one embodiment, crosslinked polyvinylpyrrolidone (crospovidone USP/NF) is an optional disintegrant for tablet or capsule disintegration, and, if present, may optionally constitute about 1 % to about 5% of the total weight of the composition.

In another embodiment, chitin is an optional disintegrant for tablet or capsule disintegration.

In still another embodiment, chitosan is an optional disintegrant for tablet or capsule disintegration.

In still another embodiment, carboxymethyl cellulose (sodium CMC) is an optional disintegrant for tablet or capsule disintegration.

In another embodiment, croscarmellose sodium is a disintegrant for tablet or capsule disintegration, and, if present, may optionally constitute about 0.2% to about 10%, about 0.2% to about 7%, or about 0.2% to about 5%, of the total weight of the composition.

Various embodiments described herein optionally comprise one or more pharmaceutically acceptable binding agents or adhesives as excipients, particularly for tablet formulations. Such binding agents and adhesives may impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, for example, either individually or in combination, acacia; tragacanth; sucrose; gelatin: glucose; starches such as, for example, pregelatinized starches (e.g., National™ 1511 and National™ 1500); celluloses such as, for example, methylcellulose and carmellose sodium (e.g., Tylose™); alginic acid and salts of alginic acid; magnesium aluminum silicate; PEG; guar gum; polysaccharide acids; bentonites; povidone, for example povidone K-15, K-30 and K 29/32: polymethacrylates; HPMC; hydroxypropylcellulose (e.g., Klucel™); and ethylcellulose (e.g., Ethocel™). Such binding agents and/or adhesives, if present, may constitute in total about 0.5% to about 25%, about 0.75% to about 15%, or about 1% to about 10%, of the total weight of the composition.

Compositions described herein optionally comprise one or more pharmaceutically acceptable wetting agents as excipients. Illustrative examples of surfactants that can be used as wetting agents in various compositions include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (e.g., Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (e.g., Tween™ 80 of ICI), propylene glycol fatty acid esters, for example propylene glycol laurate (e.g., Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, may constitute in total about 0.25% to about 15%, about 0.4% to about 10%, or about 0.5% to about 5%, of the total weight of the composition.

Compositions described herein optionally comprise one or more pharmaceutically acceptable lubricants (including anti-adherents and/or glidants) as excipients. Suitable lubricants include, for example, either individually or in combination, glyceryl behapate (e.g., Compritol™ 888); stearic acid and salts thereof, including magnesium (magnesium stearate), calcium and sodium stearates; hydrogenated vegetable oils (e.g., Sterotex™); colloidal silica; talc; waxes; boric acid; sodium benzoate; sodium acetate: sodium fumarate; sodium chloride; DL-leucine; PEG (e.g., Carbowax™ 4000 and Carbowax™ 6000); sodium oleate; sodium lauryl sulfate; and magnesium lauryl sulfate. Such lubricants, if present, may constitute in total about 0.1 % to about 10%, about 0.2% to about 8%, or about 0.25% to about 5%, of the total weight of the composition.

Suitable anti-adherents include, for example, talc, cornstarch, DL-leucine, sodium lauryl sulfate and metallic stearates. Talc is a anti-adherent or glidant used, for example, to reduce formulation sticking to equipment surfaces and also to reduce static in the blend. Talc, if present, may constitute about 0.1% to about 10%, about 0.25% to about 5%, or about 0.5% to about 2%. of the total weight of the composition.

Glidants can be used to promote powder flow of a solid formulation. Suitable glidants include, for example, colloidal silicon dioxide, starch, talc, tribasic calcium phosphate, powdered cellulose and magnesium trisilicate.

Compositions described herein can comprise one or more flavoring agents, sweetening agents, and/or colorants. Flavoring agents useful in the present embodiments include, for example, acacia syrup, alitame, anise, apple, aspartame, banana, Bavarian cream, berry, black currant, butter, butter pecan, butterscotch, calcium citrate, camphor, caramel, cherry, cherry cream, chocolate, cinnamon, citrus, citrus punch, citrus cream, cocoa, coffee, cola, cool cherry, cool citrus, cyclamate, cylamate, dextrose, eucalyptus, eugenol, fructose, fruit punch, ginger, glycyrrhetinate, glyeyrrhiza (licorice) syrup, grape, grapefruit, honey, isomalt, lemon, lime, lemon cream, MagnaSweet®, maltol, mannitol, maple, menthol, mint, mint cream, mixed berry, nut, orange, peanut butter, pear, peppermint, peppermint cream, Prosweet® Powder, raspberry, root beer, rum, saccharin, safrole, sorbitol, spearmint, spearmint cream, strawberry, strawberry cream, stevia, sucralose, sucrose, Swiss cream, tagatose, tangerine, thaumatin, tutti fruitti, vanilla, walnut, watermelon, wild cherry, wintergreen, xylitol, and combinations thereof, for example, anise-menthol, cherry-anise, cinnamon-orange, cherry-cinnamon, chocolate-mint, honey-lemon, lemon-lime, lemon-mint, menthol-eucalyptus, orange-cream, vanilla-mint.

Sweetening agents that can be used in the present embodiments include, by way of illustrative example, acesulfame potassium (acesulfame K), alitame, aspartame, cyclamate, cylamate, dextrose, isomalt, MagnaSweet®, maltitol, mannitol, neohesperidine DC, neotame, Prosweet® Powder, saccharin, sorbitol, stevia, sucralose, sucrose, tagatose, thaumatin, xylitol.

The foregoing excipients can have multiple roles. For example, starch can serve as a filler as well as a disintegrant. The classification of excipients listed herein is merely for illustration.

### Pharmaceutical Dosage Forms

The invention can be a pharmaceutical, nutritional, medical food or dietetic preparation. The invention can be in the form of, for example, a liquid, powder, bar, cookie, dessert, concentrate, paste, sauce, gel, emulsion, tablet, capsule to provide the daily dose of the bioactive components either as a single dose or in multiple doses. The compounds may also be administered parenterally, either directly, or formulated in various oils or in emulsions or dispersions, using either intravenous, intraperitoneal, intramuscular or subcutaneous routes. The products can be packaged by applying methods known in the art, to keep the product stable during shelf life and allow easy use or administration.

In various embodiments, compositions can be formulated as oral solid, liquid, or semi-solid dosage forms, in one embodiment, such compositions are in the form of discrete dosage forms, dose units or dosage units (e.g., tablet, capsule). The terms "dosage form", "dose unit" and/or "dosage unit" herein refer to a portion of a pharmaceutical composition that contains an amount of a therapeutic agent suitable for a single administration to provide a therapeutic effect. Such dosage units may be administered one to a small plurality (i.e. 1 to about 4) of times per day, or as many times as needed to elicit a therapeutic response. A particular dosage form can be selected to accommodate any desired frequency of administration to achieve a specified daily dose. Typically one dose unit, or a small plurality (i.e. up to about 4) of dose units, provides a sufficient amount of the active agent(s) to result in the desired response or effect.

In another embodiment, a single dosage unit, be it solid or liquid, comprises a therapeutically and/or prophylactically effective amount of the active agent(s). The term "therapeutically effective amount" or "therapeutically and/or prophylactically effective amount" as used herein refers to an amount of compound or agent that is sufficient to elicit the required or desired therapeutic and/or prophylactic response, as the particular treatment context may require.

It will be understood that a therapeutically and/or prophylactically effective amount of an agent for a subject is dependent, inter alia, on the body weight of the subject. A "subject" herein to which a therapeutic agent or composition thereof can be administered includes a human subject of either sex and of any age, and also includes any nonhuman animal, particularly a domestic or companion animal, illustratively a cat, dog or a horse.

### Liquid Dosage Forms

Compositions described herein are in the form of liquid dosage forms or units. Illustrative examples of suitable liquid dosage forms include solutions, suspensions, elixirs, syrups, emulsions, and gels.

In one embodiment, an oral liquid dosage form was prepared according to the following formula:

**Example 11**

| **Ingredient** | **Approx. Amount (mg) of Total Daily Dose** |
|---|---|
| EPA | 500-2500 |
| DHA | 1500-7500 |
| LA | 1400-6600 |
| GLA | 700-3300 |
| Other omega-3 PUFAs | 300-2400 |
| MUFAs | 80-2000 |
| SFAs | 150-1000 |
| Gamma-tocopherol | 100-1000 |
| Vitamin A (beta-carotene) | 0-3 |
| Vitamin E | 0-50 |
| **Total** | 4730-26353 |

### Storage Stability

In one embodiment, compositions are in the form of a liquid that is ultimately to be administered to a subject. Compositions of the disclosure are believed to exhibit improved storage stability.

### Administration and Bioavailability

In one embodiment compositions of the disclosure are suitable for immediate absorption and therapeutic effect. The preparations according to the invention can be used in the treatment and/or prevention specifically of MS. but it is also possible to be used for other neurodegenerative and/or autoimmune diseases or syndromes, it is also possible to be beneficial for spinal cord injury recovery and for stimulation of myelin formation.

### Veterinary Applications

It will be understood that where a disorder of a kind calling for treatment in animals arises, the invention while described primarily in terms of human medicine and treatment is equally applicable in the veterinary field.

### Treatment of Neurologic Disorders and Autoimmune Disease

As further described herein, the present invention, for example, employs the concomitant oral administration of EPA, DHA, LA and GLA. The formulation may further comprise Vitamin A, gamma-tocopherol and Vitamin E. Without being bound by theory, it is believed that the GLA component promotes phosphorylation and the incorporation of DHA into cell membranes, assisting in myelin production (where DHA is the major fatty acid myelin constituent). The combination facilitates the normalization of PUFA concentration within the immune cell's membrane and their function. Additionally, the LA converts to dihomo-gamma linolenic acid (DGLA), which up-regulates prostaglandin production. Prostaglandins have well-known anti-inflammatory properties. LA is a building block of lecithin (di-LA-phosphatidyl choline), which is another molecule essential for myelin composition.

By employing the high doses of the agents described herein, it is postulated that the present invention prevents excess amounts of arachidonic acid (AA) from being incorporated into the cell membranes. When less AA is released from the cell membranes, the inflammatory process is not so exaggerated. Additionally, excess amounts of the specific PUFAs of the invention will competitively inhibit the enzymatic pathways that AA is using to exert its inflammatory properties.

The combination of the specific PUFAs together with gamma-tocopherol optimizes the activity of the PUFA because gamma tocopherol acts on ROS and on the genes regulating the inflammatory process. Indeed, the therapeutic combinations of the present invention facilitate the incorporation of gamma-tocopherol in the cell membrane. This results in an extended action of gamma-tocopherol as its elimination from the body is slowed.

The ingredients of the formulation are believed to act additively or synergistically to promote and/or trigger the metabolic cascades leading to reduction of demyelination, promotion of remyelination and promotion of neuroprotection in MS and other neurodegenerative diseases. By employing the high doses of the agents described herein and through all synergistic and/or additive abilities of the formulation ingredients, it is postulated that the present invention is superior to prior treatments because it is the only one able to prevent and/or positively influence and/or treat MS and/or other neurodegenerative diseases pathogenic processes such as the iron deposits in the brain as a result of poor blood circulation due to chronic cerebrospinal venous insufficiency (CCSVI). The present invention is also able to prevent and influence the CCSVI as a primary event; through the ability of its constituent ingredients and composition formulations to (a) affect and/or prevent and/or regulate lipoprotein composition, expression of adhesion molecules and other pro-inflammatory factors, and the thrombogenicity associated with atherosclerosis development; (b) affect and/or prevent and/or regulate the persistent inflammatory-proteinase activity that leads to advanced chronic venous insufficiency (CVI) and ulcer formation resulting from complex interplay of sustained venous hypertension, inflammation, cytokine and matrix metalloproteinase (MMP) activation, and altered cellular function; (c) prevent and/or regulate iron induced endothelial damage at the level of blood-brain-barrier further leading to increased inflammation and neurodegeneration; (d) prevent and/or regulate venous outflow obstruction and venous reflux in the central nervous system resulting in pathological iron depositions leading to inflammation and neuradegeneration; (e) prevent and/or regulate inflammation-associated proteins (cytokines) that disturb the mechanisms regulating iron levels in the blood, that in turn can have impact on the immune system, since both iron deficiency and iron overload may influence the proliferation of B and T lymphocytes; (f) help reducing arterial disease and normalize the prothrombotic state by a reduction in platelet activation, a lowering of plasma triglycerides and coagulation factors and/or a decrease in vascular tone and/or by dietary effect on hemostatic and lipid factors involved in transcription regulation of multiple genes, perhaps in a subject-dependent manner; and (g) prevent and/or regulate atherosclerosis by the enhancement of high-density lipoprotein-cholesterol levels and the impairment of low-density lipoprotein-cholesterol levels, the low-density lipoprotein susceptibility to oxidation, cellular oxidative stress, thrombogenicity and atheroma plaque formation by the use of specific MUFA and the increase of high-density lipoprotein cholesterol levels and the reduction of thrombogenicity, atheroma plaque formation and vascular smooth muscle cell proliferation by the use of the specific PUFA.

When administered to MS patients, the compositions result in a statistically significant reduction of annual relapse rate, reduction of relapse frequency, a statistically significant reduction of disability progression (i.e., reduction of the probability of one point increase on the Expanded Disability Status Scale (EDSS)), and the reduction of development of new or enlarging T-2 lesions of the brain in Magnetic Resonance Imaging (MRI) scans without any significant side effects.

Indeed, the present invention results in superior treatment of MS over the prior art. It can prevent the disease from occurring in a subject, which may be predisposed to the disease but has not yet been diagnosed; it may arrest its development; and it can cause regression and even eliminate the disease or its symptoms.

In various embodiments, the present disclosure provides for therapy of various diseases and disorders. Such diseases and disorders include, *inter alia,* neurologic disorders and, in particular, neurodegenerative diseases such as multiple sclerosis (MS). In addition, the present disclosure provides for therapy of autoimmune diseases. Further, the invention herein may be useful to treat psychiatric disease, inflammatory diseases or disorders, cardiovascular diseases, epilepsy and epileptogenesis.

The term "therapy" as used herein refers to treatment and/or prevention of a disorder or disease, such as a neurologic disorder or autoimmune disease.

The term "treat" or "treatment" as used herein refers to any treatment of a disorder or disease, and includesfor example, preventing the disorder or disease from occurring in a subject that may be predisposed to the disorder or disease but has not yet been diagnosed as having the disorder or disease; inhibiting the disorder or disease, for example, arresting the development of the disorder or disease; relieving the disorder or disease, for example, causing regression of the disorder or disease; or relieving the condition caused by the disease or disorder, for example, stopping the symptoms of the disease or disorder.

The term "prevent" or "prevention", in relation to a disorder or disease, means preventing the onset, of the disorder or disease development if none had occurred, or preventing further disorder or disease development if the disorder or disease was already present.

Compositions of the present disclosure can be in the form of an orally deliverable dosage unit. The terms "oral administration" or "orally deliverable" herein include any form of delivery of a therapeutic agent or a composition thereof to a subject wherein the agent or composition is placed in the mouth of the subject, whether or not the agent or composition is swallowed. Thus, "oral administration" includes buccal and sublingual as well as esophageal administration.

The foregoing lists of disorders or diseases are meant to be illustrative and not exhaustive as a person of ordinary skill in the art would recognize that there are other disorders or diseases to which the embodiments of the present disclosure could treat and/or prevent.

In one embodiment, compositions are for treating and/or preventing a disorder or disease by administering a pharmaceutical composition comprising therapeutically effective amounts of EPA, DHA, GLA and LA.

In yet another embodiment, compositions are for treating and/or preventing a disorder or disease by orally administering a pharmaceutical composition comprising therapeutically effective amounts of EPA, DHA, GLA and LA, and optionally, gamma-tocopherol, Vitamin E and Vitamin A.

In another embodiment, compositions are for treating and/or preventing a disorder or disease by orally administering a pharmaceutical composition to a subject in need thereof, comprising one of the formulations exemplified above.

As used herein, "synergism", "synergy", "synergistic effect", or "additive effect" refers to the enhancement in action or effect of two or more particular drugs used together compared to the individual effects of each drug when used alone. Without being bound to theory, it is believed that the ingredients of the inventive formulations exhibit synergism in treating the subject disease or disorder.

The use of the present invention as an adjuvant to conventional existing drugs for all these diseases and syndromes is believed to provide improved outcomes. Accordingly, the present invention can be administered simultaneously with other medications.

The formulations described herein reduce active disease progression, are able to activate remyelination but also maintain key membrane lipid components that are otherwise specifically significantly reduced in MS, suggesting a correction of a metabolic defect, not otherwise effectively treated by any existing and/or available therapy.

It is possible to use this specific intervention as monotherapy as a first-line treatment or as soon as there are indications of an ongoing neurological disease (prodromal phase). The use of the invention for prevention by populations at risk it is also possible.

Such formulation has the benefit of creating the conditions necessary for lesion formation inhibition and for lesion repair and remyelination, something that has not been achieved with any medication previously provided for MS.

### CLINICAL EXAMPLES

### Introduction

A randomized, double-blind, placebo-controlled trial was conducted to evaluate the safety and efficacy of three formulations against placebo in MS patients (Relapsing Remitting (RR)). MS is a chronic inflammatory disease of the central nervous system (CNS). It most commonly affects individuals between the ages of twenty and forty, and in higher numbers in women than men (3 to 2). In MS, a loss of the nerves' axon coating myelin prohibits the nerve axons from efficiently conducting action and synaptic potentials. As a result of oligodendrocyte (myelin producing cells) damage, a subsequent axonal demyelination is a hallmark of this disease. Scar tissue (plaques or lesions) forms at the points where demyelination occurs in the brain and spinal cord. Different pathogenic mechanisms, for example, immune-mediated inflammation, oxidative stress and excitotoxicity, are involved in the immunopathology of MS. Polyunsaturated fatty acid (PUFA) and antioxidant deficiencies along with decreased cellular antioxidant defense mechanisms have been observed in MS patients. Furthermore, antioxidant and PUFA treatment in experimental allergic encephalomyelitis (EAE), an animal model of MS, decreased the clinical signs of disease. Low-molecular-weight antioxidants may support cellular antioxidant defenses in various ways, including radical scavenging, interfering with gene transcription, protein expression, enzyme activity and by metal chelating and quenching. PUFAs are able to control immune-mediated inflammation through their incorporation in immune cells but also may affect cell function within the CNS. Both dietary antioxidants and PUFAs have the potential to reduce disease symptoms severity and activity by targeting specific pathogenic mechanisms and supporting recovery in MS (remyelination).

The present study is unique because: (a) it is the only investigation testing formulations of specific PUFAs along with γ-tocopherol in MS patients, (b) the quantity/quality of the formulation ingredients used are significantly different than any previous reported work; (c) all drop outs are continued to be clinically followed; (d) the design of the study is completely different than any previous reported study of PUFAs with inclusion and exclusion criteria; (e) the concept of the study follows the U.S. Food and Drug Administration (FDA) standards for drug clinical trials and the International Conference of Harmonization (ICH) guidelines and the Committee for Medicinal Products for Human Use (Guideline on Clinical Trials in Small Population); (f) the design diminishes all possible bias; (g) new, more than two, statistical methods are used for the analysis of results for better conclusions; (h) multiple end points and multiple comparison analyses are performed to minimize false outcomes and statistically power the results; (i) all commonly used methods for the analysis of relapses and disability progression of MS patients are also used; and (j) the design satisfies the internationally accepted guidelines for MS treatment efficacy clinical project rules presented by CONSORT 2010 (check list), and is in agreement with the guidelines for Good Clinical Practice (GCP). It is the first known study that evaluates interventions based on the complex multifactorial nature of the disease composed by systems medicine through systems biology and nutritional systems biology philosophy.

The formulations administered in the study were as follows:

### Intervention Formula A.

Oral solution administered in a daily dose of about 19.5 ml daily for 30 months: The solution contains approximately:
EPA 1650 mg/dose
DHA 4650 mg/dose
GLA 2000 mg/dose
LA 3850 mg/dose
Other omega-3 PUFAs 600 mg/dose, comprising:
   Alpha-linolenic acid (C18:3n-3) 37 mg/dose
   Stearidonic acid (C18:4n-3) 73 mg/dose
   Eicosatetraenoic acid (C20:4n-3) 98 mg/dose
   Docosapentaenoic acid (C22:5n-3) 392 mg/dose
MUFAs, comprising:
   18:1-1300 mg/dose
   20:1-250 mg/dose
   22:1-82 mg/dose
   24:1-82 mg/dose
SFAs, comprising:
   19:0-160 mg/dose
   16:0-650 mg/dose
Vitamin A 0.6 mg/dose
Vitamin E 22 mg/dose
Citrus extract qs to 19.5, ml

### Intervention Formula B

Oral solution administered in a daily dose of about 19.5 ml for daily for 30 months. The solution contains approximately:
EPA 1650 mg/dose
DHA 4650 mg/dose
GLA 2000 mg/dose
LA 3850 mg/dose
Other omega-3 PUFAs 600 mg/dose, comprising:
   Alpha-linolenic acid (C18:3n-3) 37 mg/dose
   Stearidonic acid (C 18:4n-3) 73 mg/dose
   Eicosatetraenoic acid (C20:4n-3) 98 mg/dose
   Docosapentaenoic acid (C22:5n-3) 392 mg/dose
MUFAs, comprising:
   18:1-1300 mg/dose
   20:1-250 mg/dose
   22:1-82 mg/dose
   24:1-82 mg/dose
SFAs, comprising:
   18:0-160 mg/dose
   16:0-650 mg/dose
Vitamin A 0.6 mg/dose
Vitamin E 22 mg/dose
Gamma-tocopherol 760 mg/dose
Citrus extract qs ad to 19.5 ml

### Intervention Formula C

Oral solution administered in a daily dose of 19.5 ml for 30 months. The solution contains approximately:
Gamma-tocopherol 760 mg/dose
Pure virgin olive oil 16137 mg
Citrus extract qs ad to 19.5 ml

### Intervention Formula D (Placebo)

Oral solution administered in a daily dose of 19.5 ml for 30 months. The solution contains pure virgin olive oil (16930 mg) and citrus extract.

### METHODS

### Patients

Eighty patients that represent about 20% of the total MS population in Cyprus with RR MS eligible for treatment were enrolled in this four (4) parallel treatment arm design clinical trial study at the Cyprus Institute of Neurology and Genetics (single centered study) in July 2007. All patients gave written informed consent. The period from enrollment until December 31, 2007 was used for the normalization period (as described below) and the study extended until December 31,2009.

The study protocol was developed by the investigators and it was approved by Cyprus National Bioethics committees according to European Union (EU) guidelines. Study data were collected by the investigators and were saved by the Helix Incubator Organization of Nicosia University (legal authority organization assigned by the Government) that also kept the blinded codes of the study. Statistical analysis was blindly analyzed by statisticians at the University of Cyprus and Ioannina, School of Medicine, Greece.

Enrollment was limited to men and women who were between the ages of 18 and 65 years and had a diagnosis of RR MS; who had a score of 0.0 to 5.5 on the Expanded Disability Status Scale (EDSS), a rating that ranges from 0 to 10, with higher scores indicating more severe disease; who had undergone magnetic resonance imaging (MRI) showing lesions consistent with multiple sclerosis; who had had at least one medically documented relapse within the 24 months before beginning the study; and who had been receiving approximately the same medical treatment or no treatment during the two years before enrollment. Patients were excluded because of prior immunosuppressants or monoclonal antibodies therapy, pregnancy or nursing, the presence of progressive multiple sclerosis, or any severe disease other than multiple sclerosis compromising organ function. Additional exclusion criteria included the following: consumption of any additional food supplement formula, vitamin of any type or any form of PUFA (omega-3 or omega-6) during the trial. Patients known to have a history of recent drug or alcohol abuse were also excluded. The lost to follow patients (with complete missing data) were excluded by protocol from the intent to treat analysis. Any patient that changed type of the disease, i.e., from RR MS to secondary progressive MS, during the study, were also excluded by protocol from the analysis to eliminate dramatic changes of the phenomenon of increasing disability without relapsing. If anyone was using any other supplement of any type at any time during the study was a reason for permanent discontinuation from the study. All the rest of drop outs (excluding the above three categories) continued to be medically followed for the intention to treat analysis. The drop outs, at any time and even the drop outs that never received the assigned interventions were followed like all other participants. Patients were strongly encouraged to remain in the study for follow-up assessments even if they had discontinued the assigned study intervention formula.

### Study Design and Randomization

### Randomization

Patients were equally randomly assigned to four intervention groups (three for the intervention groups and one for placebo) in a 1:1:1:1 ratio by flipping a coin, stratified by gender (women to men, 3:1). The randomization scheme was generated and securely stored by Helix Incubator Organization of Nicosia University (HIONU).

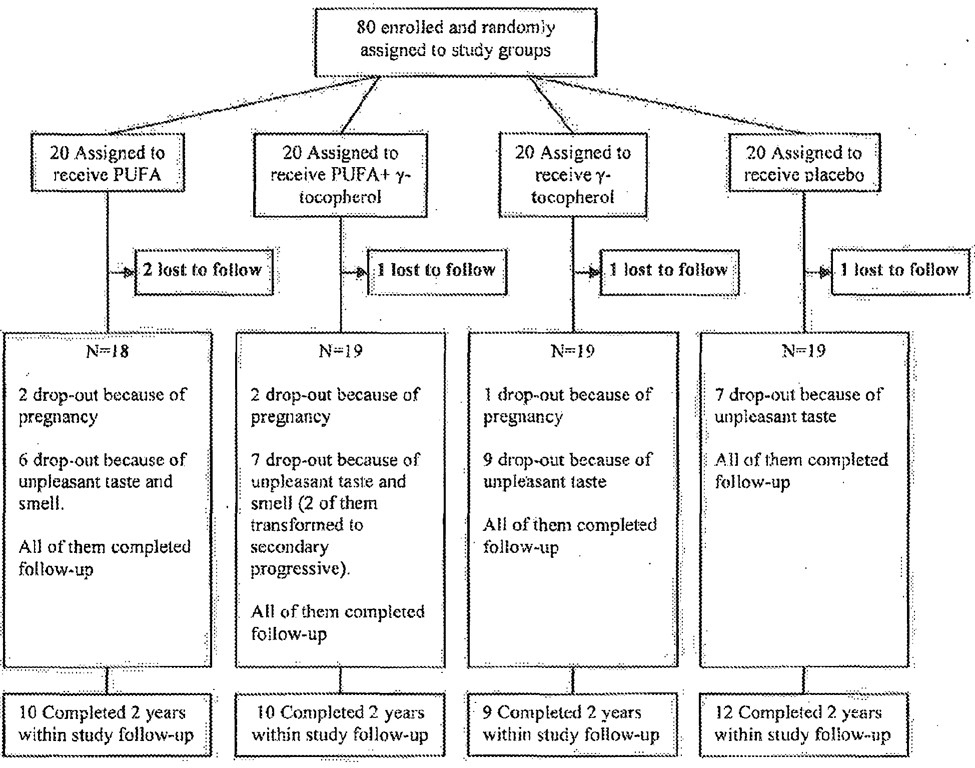

Group A was administered a composition of Intervention Formula A described above at a dose of 19.5 ml for 913 days (30 months), Group B was administered a composition consisting of Intervention Formula B (PLP10) described above at a dose of 19.5 ml for 913 days (30 months); Group C was administered a composition of Intervention Formula C described above at a dose of 19.5 ml for 913 days (30 months); and Group D, the control group, was administered a composition of Intervention Formula D described above at a dose of 19.5 ml for 913 days (30 months). All formula syrups were aromatized with citrus extract aroma. All different formulas and placebo were liquids and had identical appearance and smell. The bottles containing the syrup were labeled (by the pharmacist who was also blinded for the trial) with medication code numbers that were unidentifiable for patients as well as investigators.

All study personnel and patients involved in the conduct of the study as well as the statistician and the investigators were unaware of treatment assignments throughout the study. Group A consisted of 20 patients (15 female and 5 male) with RR MS. They had a mean age of 37.95 years, a mean disease duration of 9.00 years, an annual relapse rate (range) of 1.17 (1 to 6), a mean (range) baseline expanded disability status scale (EDSS) score of 2.52 (1.0 to 5.5) and 55% were on conventional treatment (disease modified treatment (DMT)) and 45 % were on no DMT. Group B consisted of 20 patients (15 female and 5 male) with RR MS. They had a mean age of 36.90 years, a mean disease duration of 8.55 years, an annual relapse rate (range) of 1.21 (1 to 7), a mean (range) baseline EDSS score of 2.15 (1.0 to 4.0) and 45% were on conventional treatment (DMT) and 55 % were on no DMT. Group C consisted of 20 patients (15 female and 5 male) with RRMS participating. They had a mean age of 37.65 years, a mean disease duration of 8.55 years, a annual relapse rate (range) of 1.16 (1 to 6), a mean (range) baseline EDSS score of 2.42 (0.0 to 5.0) and 60% were on conventional treatment (DMT) and 40 % were on no DMT. Group D consisted of 20 patients (15 female and 5 male) with RR MS. They had a mean age of 38.10 years, mean disease duration of 7.65 years and annual relapse rate (range) of 1.05 (1 to 4), a mean (range) baseline EDSS score of 2.39 (1.0 to 4.0) and 50% were on conventional treatment (DMT) and 50 % were on no DMT.

**Table 1. Demographic and Pre-Study Baseline Characteristics for Total Study Population by Treatment Arm.**

| Characteristics | Group A | Group B | Group C | Placebo | PValue |
|---|---|---|---|---|---|
| | (n=20) | (n=20) | (n-20) | (n=20) | |
| Sex | | | | | |
| Male | 5 (25%) | 5 (25%) | 5 (25%) | 5 (25%) | |
| Female | 15 (75%) | 15 (75%) | 15 (75%) | 15 (75%) | 1.000 |
| Age (yr) | | | | | |
| Mean | 37.95 | 36.90 | 37.65 | 38.10 | 0.982 |
| Range | 22-65 | 25-61 | 24-54 | 21 - 58 | |
| Pre-study disease duration (yr) | | | | | |
| Mean | -9.00 | 8.55 | 8.55 | 7.65 | 0.908 |
| Range | 2-37 | 2-20 | 3-24 | 2-25 | |
| Pre-study Relapses rate | | | | | |
| Mean | 2.33 | 2.41 | 2.31 | 2.10 | .0946 |
| Range | 1-6 | 1-7 | 1-6 | 1-4 | |
| Annual relapse rate | 1.17 | 1.21 | 1.16 | 1.05 | |
| Study Base line EDSS score | | | | | |
| Mean | 2.52 | 2.15 | 2.42 | 2.39 | 0.775 |
| Range | 1.0-5.5 | 1.0-4.0 | 0.0-5.0 | 1.0-4.0 | |

There were no statistical significant differences between the four Groups in regard to the epidemiological data (see Table 1 p- values). No differences were found to be present between the conventional treatment data between all treatment Groups.

### Study Design

EPA and DHA essential fatty acids are shown to be constituents of most cell membranes and neurons and crucial for different cellular and molecular physiological functions, as discussed above; but are found to be dramatically decreased in patients with autoimmune neurological disorders such as MS. Our aim was to test the possible beneficial effect of EPA and DHA with or without gamma-tocopherol but in the presence of LA, GLA, and Vitamins A and E when these molecules are used as pharmaceutical preparation/nutritional ingredients for medical use in a formula intervention with specific ratio quantities and quality; and of normalizing the EPA and DHA levels in these patients by a focused. efficacy clinical trial with specific primary end points on relapse rate and secondary end points on the disability progression when used as adjuvant therapy and as monotherapy for MS patients. The study consisted of a normalization (pre-treatment) phase. The patients were on normalization from the time of enrollment, July 2007, to December 31, 2007. This time period interval was considered for normalization/calibration of the subjects and adaptation period since (a) the incorporation of diet PUFA on the immune system is a long time process, (b) the T lymphocytes are produced in very slow rate in adulthood and even much slower in older people, (c) in supplementation clinical trials it is observed that the experimental subjects need 4-6 months to calibrate their completely different diet habits and they need time to get used to the taste, smell and intake time, (d) dietary PUFA need 4 to 6 months to have pronounced influence on cytokines and eicosanoids and tumor necrosis factor-alpha production and serum soluble IL-2 receptors in peripheral blood mononuclear cells (PBMCs) of MS patients and a significant decrease in the levels of IL-1 beta and TNF-alpha, and (e) because there are reports indicating that oral PUFA diet supplements need 4-6 months to have a neurological effect in contrast to intravenous administration. We wanted to correct any probable PUFA deficiency and normalize as much as possible, so we would be able to accurately record the efficacy as a result of the interventions even though the patients under medical treatment were randomized without any significant differences within the four treatment arms, and finally (f) to eliminate any placebo effect and regression to the mean.

The method used for the confirmation of the incorporation of PUFAs in the RBCs membrane was based on a standard protocol (Fatty Acid Analysis Protocol, 2003, Institute of Brain Chemistry and Human Nutrition, London Metropolitan University). The incorporation of PUFA in RBC membrane was evaluated by Gas Chromatography (GC). Blood sample was collected from all enrolled patients at the time of enrollment, at 3 months and at every scheduled clinical assessment from the Entry Baseline to the end of the trial. Blood was also collected during relapses. The results of this study were available to the Helix Incubator for evaluation and open to the investigators after the completion of the trial so the blindness was not jeopardized. PUFA isolation, characterization and quantification were performed using the above mentioned standard protocol. In parallel to the fatty acid analyses, routine hematological and biochemical blood test analysis were regularly performed for safety , evaluation analysis. It is suggested that PUFA deficiency needs to be corrected and things be normalized as much as possible before obtaining the drug effect.

The two year pre-entry data were collected from patients medical file records. The 24 month period between January 1, 2008 and December 31, 2009, is defined as the actual treatment period. The positive effects (improvement of relapse rate and actual effect on immune system and CNS) from Specific PUFA diet require 4-6 months to come into an effect

The four intervention formulas were used as cocktail regimens of nutritional agents for medical use and were taken orally. This study is a proof of concept, per-protocol efficacy specific trial with inclusion of intent-to-treat analysis.

We considered disability worsening when patient worsened by at least 1.0 EDSS point between two successive clinical evaluations; stable when they remained the same or increased or decreased by 0.5 EDSS point; and improvement when decreased by 1.0 EDSS point that was sustained for 24 weeks (progression could not be confirmed during a relapse). The EDSS score for disability progression is a progressive event (all future events have an added value on the previous score (positively or negatively).

The drop outs, at any time and even the drop outs that never received the assigned interventions were followed like all other participants. The study was designed to give weight quality results and different approaches to the interpretation of the results were performed. The study was designed to end 30 months after enrolment and clinical assessments were scheduled at entry baseline, 3, 9, 15, 21 and 24 months on-treatment. Patients were also clinically assessed by the involved neurologist within 48 hours after the onset of new neurologic symptoms. The neurologist reviewed adverse or side-effects, examined patients, and made all medical decisions. The same neurologist determined the EDSS score.

Patients were able to visit the clinic or contact the neurologist at any time when a relapse was suspected, if there was any adverse event, side-effect or allergic reaction. The possibility that a single assigned neurologist was going to have a bias effect on results was actually not true since this specific study includes placebo group and another three by-side (parallel) groups; it was impossible for the neurologist to know about the treatment that each one of the patients was trialed with and within which one of the groups he was enrolled.

The primary end points were total relapses, mean number of relapses per patient at every six months from entry baseline to the study completion, and the ARR. A relapse was defined as new, or recurrent neurologic symptoms not associated with fever or infection, that lasted for at least 24 hours and accompanied by new neurologic signs. Relapses were treated with methyl-prednisolone at a dose of 1g intravenous per day, for three days and with prednisone orally at a dose of 1mg/kg of weight per day on a tapering scheme for three weeks. The key secondary end point at two years was the time to confirmed disability progression, defined as an increase of 1·0 or more on EDSS, confirmed after six months (progression could not be confirmed during a relapse). The final EDSS score was confirmed six months after the end of the study. A post-hoc analysis was performed assessing the proportion of patients free from new or enlarging T2 lesions on brain MRI scans at the end of the study for the per-protocol participants of the group receiving the highest effective intervention *vs.* placebo. Comparison was made versus the already available archival MRI scans up to three months before the enrolment date. MRI scans were performed and blinded analyzed at an MRI evaluation center. The patients continued to be followed for additional 12 months after completion of the trial and the relapses were recorded. Patients were strongly encouraged to remain in the study for follow-up assessments even if they had discontinued the assigned study intervention formula.

Safety measures were assessed from the time of enrollment until 12 months following study completion. Haematological and biochemical tests were performed at enrolment and at every 12 months, including renal and liver function tests, cholesterol, triglycerides, glucose and electrolytes.

The study had objective end points at different pre-specified times. At every six month-interval according to protocol the number of relapses and EDSS were recorded. Specifically, the study was designed so that the EDSS of each treatment arm to be analyzed according to the secondary end points and against placebo; but also, by comparing the disability progression within each treatment arm during the 24 months pre-treatment period against the disability progression during the treatment period. By the same concept, relapses of each treatment arm were analyzed according to the primary end points and against placebo; but also, by comparing the number of relapses and ARR within each treatment arm during the 24 months pre-treatment period against the number of relapses and ARR during the treatment period.

The patients were followed for an additional 12 months (until December 31, 2010) after completion of the trial (post-study) and the relapse incidences were reported. The conventional medical treatment of the patients within the groups was approximately distributed equally (see study design randomization trial design above).

### Results

### Study Population

This is a **controlled,** double-blind, randomized clinical trial that specifies definite clinical end points, in an attempt to demonstrate possible therapeutic and/or adjuvant therapeutic effects on conventional treatments of three different intervention formulas composed by the use of high dosage specific formulation and by specific structural form of omega-3 PUFA / omega-6 PUFAs, "other" omega-3, MUFA, SFA, vitamin A, vitamin E and γ-tocopherol in MS and in combinations as previously described. Among the 80 patients, 20 patients were assigned to each of three groups to receive the indicative intervention A: omega -3 PUFA / omega-6 PUFAs, "other" omega -3, MUFA, SFA, vitamin A, vitamin E, B: omega-3 PUFA /omega-6 PUFAs, "other" omega-3, MUFA, SFA, vitamin A, vitamin E and γ-tocopherol, C: γ-tocopherol with pure virgin olive oil as vehicle, and 20 to receive placebo-pure virgin olive oil. There were no significant differences in baseline characteristics between the treatment groups (Table 1). Also, there were no significant differences in baseline characteristics between the treatment groups for the patients that finish the 30 months study (all-time on-study) (Table 2).

**Table 2. Demographic and Pre-Study Baseline Characteristics of all-Time on-Study Study Population by Treatment Arm.**

| Characteristics | Group A | Group B | Group C | Placebo | Pvalue |
|---|---|---|---|---|---|
| | (n=10) | (n=10) | (n=9) | (n=12) | |
| Sex | | | | | |
| Male | 5 (50%). | 3 (70%) | 3 (66.6%) | 2(83.3%) | 0.419 |
| Female | 5 (50%) | 7 (30%) | 6 (33.3%) | 10(16.6%) | |
| Age (yr) | | | | | |
| Mean | 36.60 | 34.80 | 40.89 | 39.83 | 0.572 |
| Range | 22 - 65 | 26 - 43 | 29 - 54 | 21 - 58 | |
| | | | | | |
| Pre-study disease duration (yr) | | | | | |
| Mean | 9.70 | 8.30 | 11.33 | 8.67 | 0.807 |
| Range | 2-37 | 2-20 | 4-24 | 2-25 | |
| | | | | | |
| Pre-study Relapses rate | | | | | |
| No. Of Relapses | 22 | 27 | 16 | 20 | |
| Mean | 2.20 | 2.70 | 1.78 | 1.67 | |
| Annual Relapse Rate | 1.10 | 1.35 | 0.89 | 0.83 | 0.241 |
| | | | | | |
| Study Base line EDSS score | | | | | |
| Mean | 2.65 | 2.40 | 2.11 | 2.16 | 0.698 |
| Range | 1.0 - 5.5 | 1.0 - 4.0 | 1.0 - 4.0 | 1.0 - 3.5 | |

All parameters were count (variants, co-variants) in the statistical analysis and have been statistically adjusted so the results are absolutely not false positive exposed. The data used for result analysis at different time intervals in accordance with the study design are shown in Tables 3 to 11 below.

**Table 3. First and. Second Year Primary End Points of Relapses Rate per Patient as Determined by Clinical Results Based on Study Design of all-Time on-Study (Finished Study) Population by Treatment Arm.**

| Characteristics | Group A | | Group B | | Group C | | Placebo | |
|---|---|---|---|---|---|---|---|---|
| | (N=10) | | (N=10) | | (N=9) | | (N=12) | |
| End Point | 1y | 2y | 1y | 2y | 1y | 2y | 1y | 2y |
| No. of Relapses | 8 | 9 | 4 | 4 | 7 | 6 | 10 | 15 |
| Annual Relapse Rate | 0.8 | 0.9 | 0.4 | 0.4 | 0.8 | 0.7 | 0.8 | 1.25 |
| % Annual Relapse Rate change year to year | +12.5% | | 0% | | -12.5% | | +56.3% | |
| % Annual Relapse Rate Reduction year to year Against Placebo | **0%** | **-28%** | **-50%** | **-68%** | **-0%** | **-44%** | **N/A** | **N/A** |
| **P value** | **0.492** | | **0.014.** | | **0.179** | | | |

| | | | | | | | | |
|---|---|---|---|---|---|---|---|---|
| ly Number of Relapses during 1^{st} treatment year 2y Number of Relapses during 2^{nd} treatment year | | | | | | | | |

**Table 4. Primary End Points as Determined by Clinical Results Based on Study Design of all-Time on-Study Population by Treatment Arm.**

| Characteristics | Group A | Group B | Group C | Placebo |
|---|---|---|---|---|
| | (N=10) | (N=10) | (N=9) | (N=12) |
| | | | | |
| **Period of Study** | **0-6m** | **0-6m** | **0-6m** | **0-6m** |
| | | | | |
| No. of relapses | 3 | 4 | 1 | 4 |
| Annual Relapse Rate | 0.60 | 0.80 | 0.22 | 0.67 |
| % difference with Placebo | -10.4% | +19.4% | - 67.2% | N/A |
| | | | | |
| **Period of Study** | **0-12m** | **0-12m** | **0-12m** | **0-12m** |
| | | | | |
| No. of relapses | 8 | 4 | 7 | 10 |
| Annual Relapse Rate | 0.80 | 0.40 | 0.77 | 0.83 |
| % difference with Placebo | -3.6% | -51.8% | -7,2% | N/A |
| | | | | |
| **Period of Study** | **0-18m** | **0-18m** | **0-18m** | **0-18m** |
| | | | | |
| No. of relapses | 12 | 5 | 11 | 16 |
| Annual Relapse Rate | 0.80 | 0.33 | **0**.**82** | 0.89 |
| % difference with Placebo | -10% | -62.9% | -7.9% | N/A |
| | | | | |
| **Period of Study** | **0-24m** | **0-24m** | **0-24m** | **0-24m** |
| | | | | |
| No. of relapses | 17 | 8 | 13 | 25 |
| Annual Relapse Rate | 0.85 | 0.40 | 0.72 | 1.04 |
| % difference with Placebo | -18.3% | -61.5% | -30.7% | N/A |

**Table 5. Primary End Points as Determined by Clinical Results Based on Study Design of Annual Relapse Rate for every Six Month intervals of all-Time on-Study Population by Treatment Arm.**

| Characteristics | Group A | Group B | Group C | Placebo |
|---|---|---|---|---|
| | (N=10) | (N=10) | (N=9) | (N=12) |
| | | | | |
| **Period of Study** | **0-6m** | **0-6m** | **0-6m** | **0-6m** |
| | | | | |
| No. of relapses | 3 | 4 | 1 | 4 |
| Annual Relapse Rate | 0.60 | 0.80 | 0.22 | 0.67 |
| % difference with Placebo | -10.4% | +19.4% | - 67.2% | N/A |
| | | | | |
| **Period of Study** | **6-12m** | **6-12m** | **6-12m** | **6-12m** |
| | | | | |
| No. of relapses | 5 | 0 | 6 | 6 |
| Annual Relapse Rate | 1.00 | 0 | 1.33 | 1.00 |
| % difference with Placebo | 0% | -100% | +33% | N/A |
| | | | | |
| **Period of Study** | **12-18m** | **12-18m** | **12-18m** | **12-18m** |
| | | | | |
| No. of relapses | 4 | 1 | 4 | 6 |
| Annual Relapse Rate | 0.80 | 0.20 | 0.89 | 1.00 |
| % difference with Placebo | -20% | -80% | -11% | N/A |
| | | | | |
| **Period of Study** | **18-24m** | **18**-**24m** | **18-24m** | **18-24m** |
| | | | | |
| No. of relapses | 5 | 3 | 2 | 9 |
| Annual Relapse Rate | 1.00 | 0.60 | 0.44 | 1.50 |
| % difference with Placebo | -33.3% | -60% | -70.6% | N/A |

**Table 6. Analysis of End Points as Determined by Clinical Results Based on Study Design of all-Time on-Study Population by Treatment Arm of Within Study Time Windows with Most Annual Relapse Rate Difference Against Placebo.**

| **Characteristics** | **TIME WINDOW PERIODS WITRIN STUDY** | | | | | | |
|---|---|---|---|---|---|---|---|
| | D | E | F | G | **II** | I | J |
| **Group A** | | | | | | | |
| No.Relapses | 3 | 5 | 9 | 14 | 4 | 9 | 5 |
| | | | | | | | |
| **Group B** | | | | | | | |
| No. Relapses | 4 | 0 | 1 | 4 | 1 | 4 | 3 |
| Annaal Relapse Rate | 0.8 | 0 | 0.1 | 0.3 | 0.2 | 0.4 | 0.6 |
| % **Reduction** with Placebo | +19.4% | -100% | -90% | -75% | -80% | -68% | 60% |
| | | | | | | | |
| **Group C** | | | | | | | |
| No. Relapses | 1 | 6 | 10 | 12 | 4 | 6 | 2 |
| | | | | | | | |
| **Placebo** | | | | | | | |
| No. Rel^{y}nes | 4 | 6 | 12 | 21 | 6 | 15 | 9 |
| | | | | | | | |
| **Annual Relapse Rate** | 0.67 | 1.0 | 1.0 | 1.2 | 1.0 | 1.25 | 1.5 |

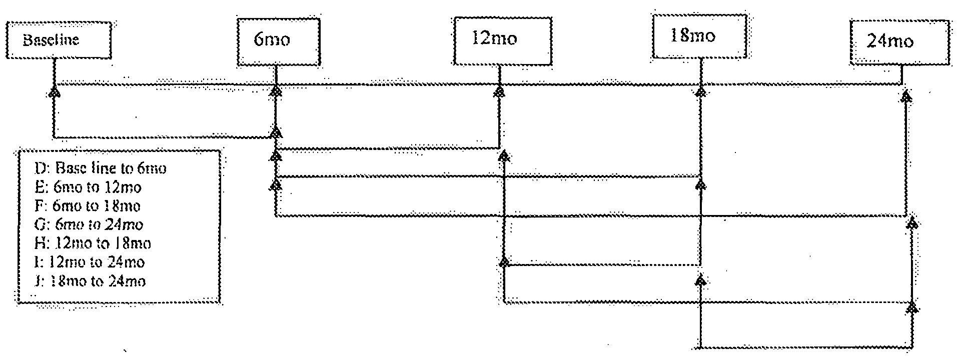

**Table 7. Comparison of Pre Study Relapse Rate to One Year within Study Relapse Rate of all-Time on-Study Population.**

| Characteristics | Group A | | Group B | | Group C | | Placebo | |
|---|---|---|---|---|---|---|---|---|
| End Point | **X** | **Y** | **X** | | **X** | **Y** | **X** | **Y** |
| | (N=10) | | (N=10) | | (N=9) | | (N=12) | |
| | | | | | | | | |
| **Total No. of relapses** | **22** | **8** | **27** | **4** | **16** | **7** | **20** | **10** |
| | | | | | | | | |
| **Annual Relapse Rate** | **1.10** | **0.80** | **135** | **0.40** | **0.88** | **0.77** | **0.83** | **0.83** |
| | | | | | | | | |
| **% Change** | **-27.3** | | **-70.4** | | **-12.5** | | **0.0%** | |

| | | | | | | | | |
|---|---|---|---|---|---|---|---|---|
| X Total number of relapses of-24 months before Entry Base Line Y Total number of relapses within one year in the Study | | | | | | | | |

**Table 8. Comparison of Pre Study Relapse Rate to Two Year within Study Relapse Rate of all-Time on-Study Population.**

| Characteristics | Group A | | Group B | | Group C | | Placebo | |
|---|---|---|---|---|---|---|---|---|
| End Point | **X** | **Y** | **X** | **Y** | **X** | **Y** | **X** | **Y** |
| | (N=10) | | (N=10) | | (N=9) | | (N=12) | |
| | | | | | | | | |
| **Total No. of relapses** | **22** | **17** | **27** | **8** | **16** | **13** | **20** | **25** |
| | | | | | | | | |
| **Annual Relapse Rate** | **1.10** | **0.85** | **1.35** | **0.40** | **0.88** | **0.72** | **0.83** | **1.04** |
| | | | | | | | | |
| **% Change** | **-22.7** | | **-70.4** | | **-18.2** | | **+25.3** | |
| **P Value** | **0.391** | | **0.0006** | | **0.303** | | **0.510** | |

| | | | | | | | | |
|---|---|---|---|---|---|---|---|---|
| X **Total number** of relapses of -24 months before Entry Base Line Y Total number of relapses within the Study | | | | | | | | |

**Table 9. Annual Relapse Rate within Each Group during 24mo Treatment and Percent Difference with Placebo of all-Time on-Study Population.**

| | GROUPA (N=10) | GROUP B (N=10) | GROUP C (N=9) | PLACEBO (N=12) |
|---|---|---|---|---|
| **Annual Relapse Rate** | **0.85** | 0.40 | 0.72 | 1.04 |
| **% Reduction** | **-18.2%** | **-61.5%** | **-30.8%** | N/A |
| **P Value** | **0.486** | **0.014** | **0.175** | |

**Table 10. Mean EDSS Progression from -24mo to Entry Baseline and From Entry Baseline to The End of the Study Within Each Group of all-Time on-Study Population.**

| | GROUP A (N = 10) | GROUP B (N=10) | GROUP C (N=9) | PLACEBO (N =12) |
|---|---|---|---|---|
| **Mean Disability Progression of patients finished the study from -24mo to entry Boseline (Pre)** | 2.05 to 2.65 | 1.70 to 2.40 | 2.11 to 2.11 | 2.08 to 2.16 |
| % **change** | +29.3% | +41.2% | 0% | +3.8% |
| **Mean Disability Progression of patients finished the study from Baseline to the end of study (Post)** | 2.65 to 3.30 | 2.40 to 2.70 | 2.11 to 2.72 | 2.16 to 3.33 |
| **% change** | **+24.5%** | **+12.5%** | **+28.9%** | **+54.2%** |
| **% Pre to Post difference** | **-16.4%** | **-69.7%** | **+28.9** | **+1326.%** |

**Table 11.**

| **Disability Increase Risk Reduction of all-Time on-Study Population** | | | | | |
|---|---|---|---|---|---|
| **GROUP** | Increase EDSS By 1 Point | | Absolute Risk Reduction (Compared to placebo) | Percentage Risk Reduction (Compared to placebo) | P Value |
| A | 4/10 | (40%) | 18% | 31% | **0.301** |
| **B** | 1/10 | (10%) | 48% | 83% | **0.049** |
| C | 2/9 | (22%) | 36% | 62% | 0.143 |
| D | 7/12 | (58%) | ---- | ---- | |

All patients, regardless of duration on study treatment, were included in the failure-time (intention to treat) analyses (Table 12 and 13 below).

**Table 12. Two Year Primary End Point of Relapses Based on Study Design as Reported by Drop Out Patients (Intention to Treat) by Treatment Arm.**

| Characteristics | Group A | | Group B | | Group C | | Placebo | |
|---|---|---|---|---|---|---|---|---|
| | (n=8) | | (n=7) | | (n=10) | | (n=7) | |
| | X | Y | X | Y | X | Y | X | Y |
| **No. of relapses** | 20 | 14 | 14 | 14 | 27 | 26 | 20 | 13 |
| **Annual Relapse Rate** | **1.25.** | **0.88** | **1.00** | **1.00** | **1.35** | **1.30** | **1.42** | **0.92** |
| **P Value** | **0.306** | | **1.000** | | **0.890** | | **0.226** | |

| | | | | | | | | |
|---|---|---|---|---|---|---|---|---|
| X: No. of relapses of pre entry period Y: No. of relapses within study | | | | | | | | |

**Table 13. Two Year Primary and Secondary End Points as Determined by Clinical Results Based on Study Design of Total Number of Patients (Intention to Treat) by Treatment Arm.**

| Characteristics | **Group A** | | **Group B** | | **Group C** | | **Placebo** | |
|---|---|---|---|---|---|---|---|---|
| | **(n=18)** | | **(n=17)** | | **(n=19)** | | **(n=19)** | |
| | X | Y | X | Y | X | Y | X | Y |
| **No. of relapses** | 42 | 31 | 41 | 22 | 43 | 39 | 40 | 38 |
| **Mean No. Relapses** | 2.33 | 1.72 | 2.41 | 1.29 | 2.26 | 2.05 | 2.11 | 2.00 |
| **Annual Relapse Rate (ARR)** | 1.17 | 0.86 | 1.21 | 0.65 | 1.13 | 1.03 | 1.06 | 1.00 |
| **ARR Reduction (Y to X)** | -26.5% | | -46.3% | | -8.8% | | -5.7% | |
| **P Value** | 0.200 | | 0.019 | | 0.579 | | 0.443 | |
| **Reduction of the ARR Of each group Compared To Placebo at the End of the Study (Y of each group** to **Y of placebo)** | -14% | | -35% | | +3% | | N/A | |
| **P Value** | 0.537 | | 0.104 | | 1.000 | | | |
| **Base line EDSS** | | | | | | | | |
| **Mean Disability Progression Of All patients from-24mo to entry Base Line (Pre)** | 2.14 to 2.53 | | 1.59 to 2.15 | | 1.97 to 2.42 | | 2.00 to 2.39 | |
| **Increase %** | +18.2% | | +35.2% | | +22.8% | | +19.5% | |
| **Mean Disability Progression Of All patients From the Entry Base line to the End Of Study (Post)** | 2.53 to 2.94 | | 2.15 to 2.47 | | 2.42 to 2.79 | | 2.39 to 2.97 | |
| **Increase %** | +16.2% | | +14.9% | | +15.3% | | +24.2% | |
| **% Pre to Post Difference** | -10.9% | | -57.7% | | -32.9% | | +24.1% | |

| | | | | | | | | |
|---|---|---|---|---|---|---|---|---|
| X: Period of -24mo to Entry Base Line Y: Period of Base Line to the End of Study | | | | | | | | |

Only 5 patients were totally lost to follow-up before their primary end point was definitively determined and it was impossible to be included in the intention to treat analysis in accordance with the study design. Two of the drop out patients were patients that later transformed from RR MS to secondary progressive MS (SPMS) during the follow up study years and were also excluded from the intention to treat analysis according to the exclusion criteria of the design (there is not a way to pre-value an MS patient when he/she is going to enter the secondary progression stage). This was the reason that they were committed the prerequisite criteria for entry into the trial.

A total of 41 (51%) patients completed all of the 30 month study, and total 39 (49%) patients either withdrew (drop out) or lost to follow. In Group A, 10 patients, in Group B, 10 patients, in Group C, 9 patients and in placebo, 12 patients completed the study. All of the patients that withdrew, except the 5 patients that were completely lost to follow and the two patients that became secondary progressive MS, completed follow-up until the end of the study. These thirty-two patients (7 placebo recipients, 8 from Group A, 7 from Group B and 10 from Group C) continued to be followed and evaluated and their result data (relapses and EDSS) were included in the intention to treat statistical analyses. A paired wise statistical analysis between groups and placebo (so the results maintain the power as designed) as well as an individual comparison of each group against placebo was followed.

### Efficacy

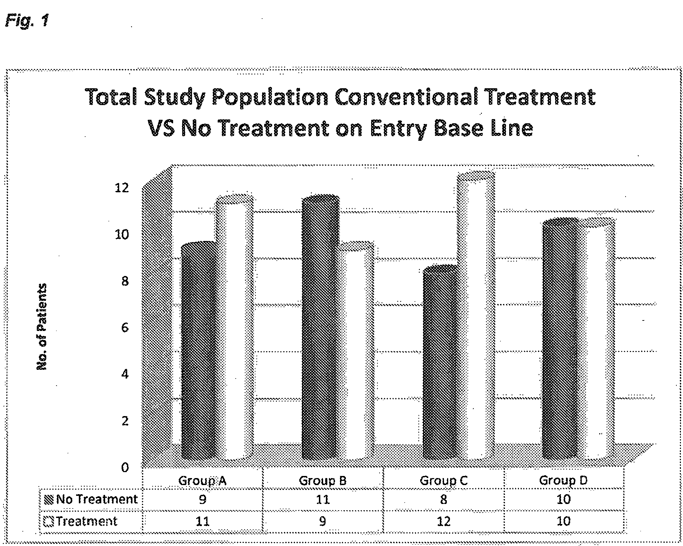

Annual relapse rate was calculated as follows: For annual relapse rate at any point, the relapse number of a patient in that time period was divided by treated days of that specific time period. These answers were multiplied by 365 (days). The annual relapse rate has been widely reported by many other authors. Although this is a standard in the field, this approach depends on the assumption that the time to a patient's first relapse is independent of the time from a patients' first relapse to their second relapse (i.e., that there are not some patients with inherently higher relapse rates than other patients). However, since this approach has been so widely used in the literature, it was necessary to include the annual relapse rate for comparability to data in other publications. Annual relapse rates also were calculated for all patients (by using the mean relapse number), using all-time on-study, in the same manner as above. Tables 3 to 9 demonstrates the relapses and mean annual relapse rate after excluding the data of the drop-out patients at different pre- and on-study time intervals according to primary and secondary end points. The trial design (above) demonstrates the percentages of the total study population that were receiving or not receiving conventional treatment at entry baseline. Figure 1 demonstrates the percentages of total study population conventional treatment vs. no treatment on entry baseline.

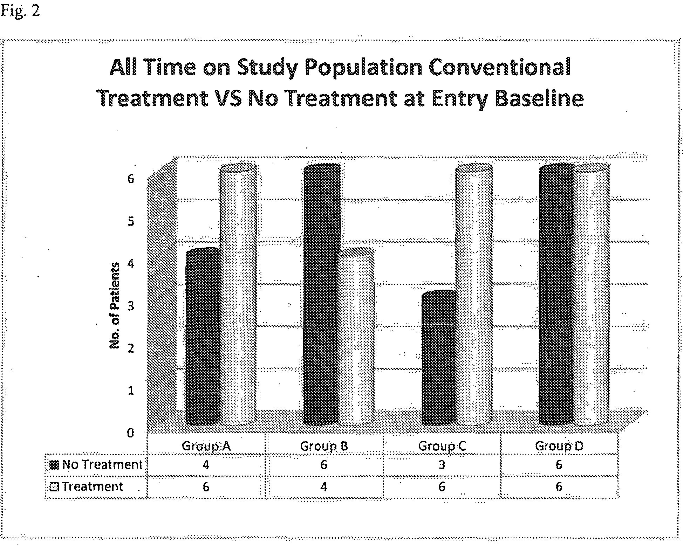

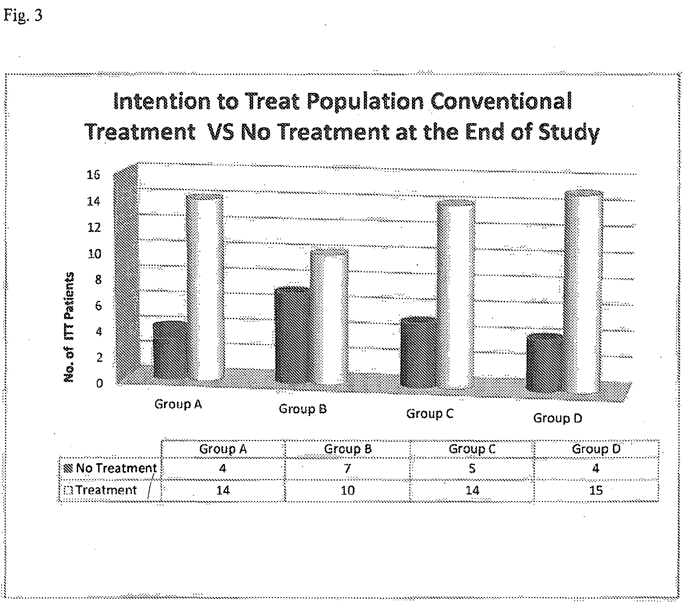

Figure 2 demonstrates the percentages of all-time on-study population that were receiving or not receiving conventional treatment at Entry Base Line. Within Group A 60% were on conventional treatment and 40 % on no treatment, within Group B 40% were on conventional treatment and 60 % on no treatment, within Group C 67% were on conventional treatment and 33 % on no treatment and within Group D 50% were on conventional treatment and 50 % on no treatment (no significant differences, p=0.799). Table 13 is for total population (including drop-outs), the intention to treat analysis. Figure 3 demonstrates the percentages of intention to treat population that were deceiving or not receiving conventional treatment at the end of the study. Within Group A 78% were on conventional treatment and 22 % on no treatment, within Group B 59% were on conventional treatment and 41 % on no treatment, within Group C 74% were on conventional treatment and 26 % on no treatment and within Group D 79% were on conventional treatment and 21 % on no treatment. From Figure 3, we can clearly realize that the conventional treatment applied on all Groups could have a significant effect on the ITT analysis (paired wise analysis) which in return could affect the ITT efficacy evaluation of Group B against placebo.

After one year of treatment, all trial Groups except placebo reduced the annualized rate of relapse (one year primary end point) (Table 3). During the first year of treatment Group A presented an annual relapse rate of 0.8, Group B an annual relapse rate of 0.4 and Group C an annual relapse rate of 0.8 as compared with 0.8 relapse per year in the placebo Group (Group D). During the second year of treatment, Group A presented an annual relapse rate of 0.9 (+12.5 percent compare to first year), Group B maintained the annual relapse rate of 0.4 relapses per year (0.0 percent compare to first year); Group C presented an annual relapse rate of 0.7 relapses per year (-12.5 percent compare to first year) and placebo increased the second year annual relapse rate to 1.25 (+ 56.3 percent increase compare to the first year). Intervention formula A had 0.0 percent annual relapse rate reduction (ARRR) in the first year and 28 percent the second year compared to placebo; Intervention formula B bad 50 percent ARRR in the first year and 68 percent the second year compared to placebo; Intervention formula C had 0.0 percent ARRR on the first year and 44 percent the second year compared to placebo.

The proportion of less or equal to one relapse per patient was significantly higher in the intervention formula B Group B than in the placebo group; 90 percent vs. 42 percent for the two year study. For Group A 50 percent vs. 42 and for Group C was 44 percent vs. 42 percent. The intervention formula B presented an Absolute Risk Reduction of 48 percentage points compared to placebo. This means that intervention formula B increases the probability of having one or less than one relapse over two year period by 114 percent compared to placebo. This observation is even stronger if we comment that in Group B at base line there were only two patients with less than 2 relapses each, two patients with two relapses each and six patients with equal or more than 4 relapses each. During the two year trial period, in Group B, nine patients ended with equal or less than one relapse and one patient had two relapses. In placebo Group, at base line there were six patients with one or less relapses each, two patients with two relapses each and four patients with three or more. During treatment, in placebo Group, five patients ended with one or less relapses each, one patient had two relapses and six patients had three or more relapses each.

Patients with two or more relapses during the period of two years before the study were: 7 out of 10 (70%) for Group A, 8 out of 10 (80%) for Group B, 6 out of 9 (67%) for Group C and 6 out of 12 (50%) in placebo Group. At the end of the study patients with two or more relapses were: 5 out of ten (50%) for Group A, 1 out of 10 (10%) in Group B, 4 out of 9 (44%) in Group C and 7 out of 12 (58%) within placebo Group. The intervention formula B presented an Absolute Risk Reduction of 70 percentage points for a patient to have two or more relapses compare to the two pre-entry years. The proportion of patients with ≤1 relapse for the two years on-study was higher in group B than in the placebo group (90% *vs*. 42%). Intervention B decreased the probability risk of a patient to have >1 relapse over two years by 83% (p=0.019) compared to placebo.

According to the above Group characteristics and from the existed knowledge of how relapse history works in relation to future relapses on MS patients, one would expect that the patients within placebo Group, that entered the study with less disease activity (6 patients with equal or less than one relapses, 2 patients with equal or less than two relapses and 4 with equal or more than three relapses), in contrast to the patients within Group B (only 2 patients equal or less than one relapse, 2 patients with equal or less than two relapses and six with equal or more than three relapses), would present the least disease activity during treatment.

In contrast to the above statement where is clearly demonstrated that although patients within Group B had entered the trial with much more baseline relapses per patient and annual relapse rate compared to placebo, after treatment, our results showed reversal of the above (the exact opposite). This outcome shows a strong, positive effect by intervention formula B (Table. 9).

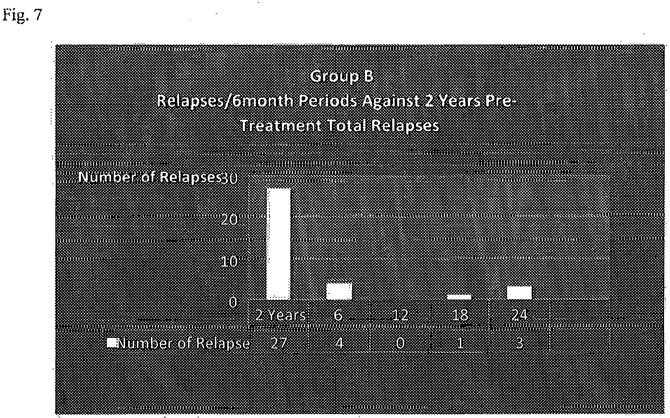

The annual relapse rate (ARR) per 6, 12, 18 and 24 month interval period during treatment in Group B compared to placebo was 0.80 vs. 0.67 in first six months (+19.4 percent difference with placebo), 0.40 vs. 0.83 during 0 to 12 months (-51.8 percent difference with placebo), 0.33 vs. 0.89 during 0 to 18 months (-62.9 percent difference with placebo) and 0.4 vs.1 .04 during 0 to 24 months (-61.5 percent difference with placebo) (Table 4) (Figure 7, relapse per 6mo period). For Group A compared to placebo was 0.60 vs. 0.67 in first six months (-10.4 percent difference with placebo), 0.80 vs. 0.83 during 0 to 12 months (-3.6 percent difference with placebo), 0.80 vs. 0.89 during 0 to 18 months (-10 percent difference with placebo) and 0.85 vs. 1.04 during 0 to 24 months (-18.3 percent difference with placebo) (Table 4). For Group C compared to placebo was 0.22 vs. 0.67 in first six months (- 67.2 percent difference with placebo), 0.77 vs. 0.83 during 0 to 12 months (-7.2 percent difference with placebo), 0.82 vs. 0.89 during 0 to 18 months (-7.9 percent difference with placebo) and 0.72 vs. 1.04 during 0 to 24 months (-30.7 percent difference with placebo) (Table 4). The annual relapse rate per 6 months interval period during treatment in Group B compared to placebo was 0.80 vs. 0.67 in first six months (+19.4 percent difference with placebo), 0.00 vs. 1.00 during second six months (-100 percent difference with placebo), 0.20 vs. 1.00 during third six months (-80 percent difference with placebo) and 0.60 vs.1.50 the last six months (-60 percent difference with placebo) (Table 5, 6). Group A compare to control showed 0.60 vs. 0.67 in first six months (-10.4 percent difference with placebo), 1.00 vs. 1.00 during second six months (0 percent difference with placebo), 0.80 vs.1.00 during third six months (-20 percent difference with placebo) and 1.00 vs. 1.50 the last six months (-33.3 percent difference with placebo) (Table 5, 6). Group C compare to control showed 0.22 vs. 0.67 in first six months (-67.2 percent difference with placebo), 1.33 vs. 1.00 during second six months (+33 percent difference with placebo), 0.89 vs. 1.00 during third six months (-11 percent difference with placebo) and 0.44 vs. 1.50 the last six months (-70.6 percent difference with placebo) (Table 5, 6).

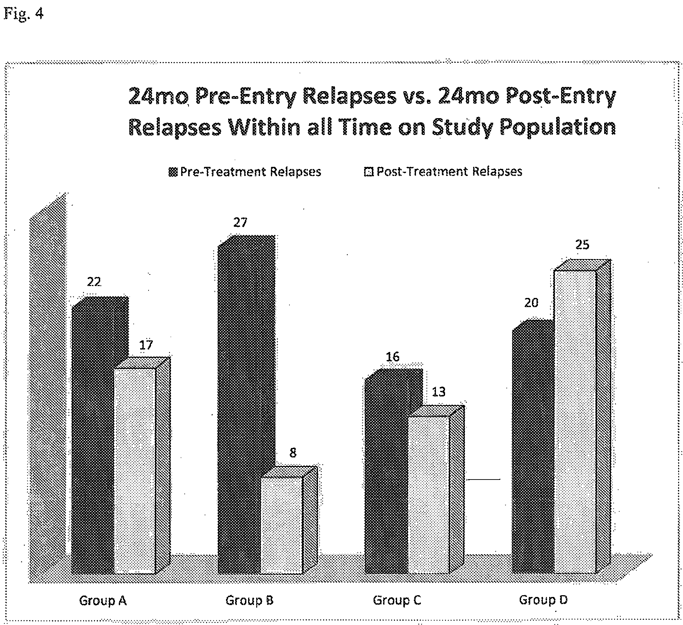

The comparison of pre-study annual relapse rate to one year within study relapse rate of finished study population is shown in Table 7. Group A showed a -27.3 percent decrease, Group B a -70.4 percent decrease and Group C -12.5 percent decrease and placebo 0.0 percent difference. Table 8 is for the comparison of two year pre-study annual relapse rate to two years within study annual relapse rate of finished study population. Group A showed -22.7 percent decrease (from 22 relapses of two years pre-entry to 17 relapses of two years within study) p=0.391 CI 95%, Group B -70.4 percent decrease (from 27 relapses of two years pre-entry to 8 relapses of two years within study) p=0.0006 CI 95%, Group C -18.2 percent decrease (from 16 relapses of two years pre-entry to 13 relapses of two years within study) p=0.303 CI 95%, and Placebo +25.3 percent increase (from 20 relapses of two years pre-entry to 25 relapses of two years within study) p=0.510 CI 95%, (Fig. 4).

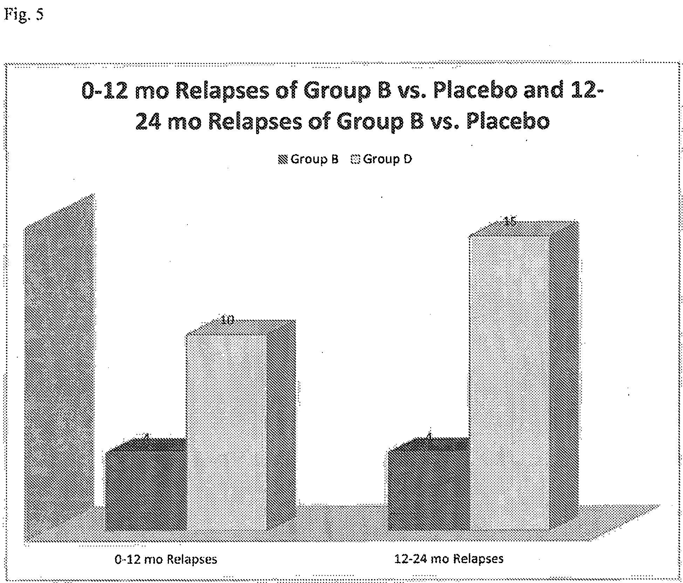

An increase in annual relapse rate is shown within placebo group with significant difference compare to Group B where relapse rate is dramatically drop down within six months and stabilized as such (Fig. 4, 5). This phenomenon most probably reflects the scientific knowledge that PUFAs needs 4-6 months to exert their clinical effects. Clearly, no placebo effect can account for these results since this is a controlled based trial and four parallel groups are treated; the first six months of the study where the placebo effect usually has an effect, can not be the case here since in this trial the first six months are used for normalization / calibration. These placebo bias effects can only be count in single group trials without control and without normalization period, where here is not valid. There is no any bias at this point of result analysis for another reason: the existence of the other three parallel treated groups involved in the study that are also included in the paired statistical analysis. As far as the number of subjects within each group we need to discuss that the 80 MS patients (n=20 per Group) within the study represent the 20 percent of the total RRMS population who were candidates for DMT treatment in Cyprus and this is a strong parameter for the statistical power of the study. When trials are appropriately designed and all appropriate scientific clinical study parameters (proposed by FDA and European Medicines Agencies (EMA)) are followed then the power of the results is with a great value. In addition, the three parallel groups in this study give dynamic comparison between groups and placebo.

The main conclusion of all different ways of result analyses is that intervention formula B is of great value with definite positive activity on MS and is statistically significant (p=0.0006, 95% confidence interval, when compared to the two years before entry in relation to placebo and p=0.014, 95% confidence interval, when it is compared to the placebo for the two years within the study). It is clear that the patients treated with intervention formula B had significantly fewer relapses. Group A had a p=0.391 when compared to the two years before entry in relation to placebo and p=0.486 when it is compared to the placebo for the two years within the study and Group C had a p=0.303 when compared to the two years before entry in relation to placebo and p=0.175 when it is compared to the placebo for the two years within the study for the same result analysis as Group B (Tables 8, 9).

As for the patients in the trial, specifically in Group B, the most percentage decrease in relapse rate against placebo was observed between (a) the 6^{th} to 12^{th} month within the study (100 percent decrease); (b) between the 6^{th} to 18^{th} (90 percent decrease) and (c) between the 6^{th} and 24^{th} month within the study (75 percent decrease) (Table 6). These time period windows within the Group B showed 0.00, 0.10 and 0.30 annual relapse rate respectively. This means that in Group B during period 6^{th} to 12^{th} month all patients were relapse free, during period 6^{th} to 18^{th} months only 1 patient patients out of 10 had 1 relapse in comparison to placebo where each one of the patients had 1 relapse during 6^{th} to 12^{th} months period, during 6^{th} to 18^{th} months period once again each one of the patients had 1 relapse and during 6^{th} to 24^{th} months period each one of the patients had 1.2 relapses. Not one of the other two parallel Groups (Group A and C) showed this long time free relapse intervals. These result analyses give the conclusion that formula B has the maximum effect after the first six months within the study and from that point on, there is a maximum effect until the end; i.e. the annual relapse rate is stabilized.

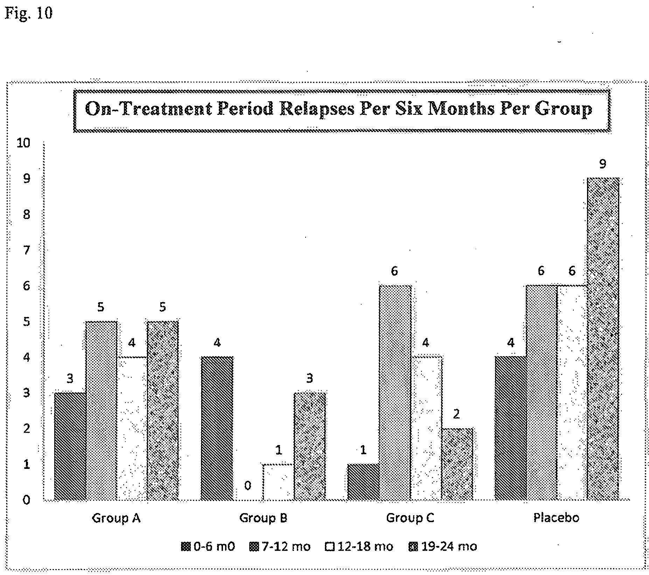

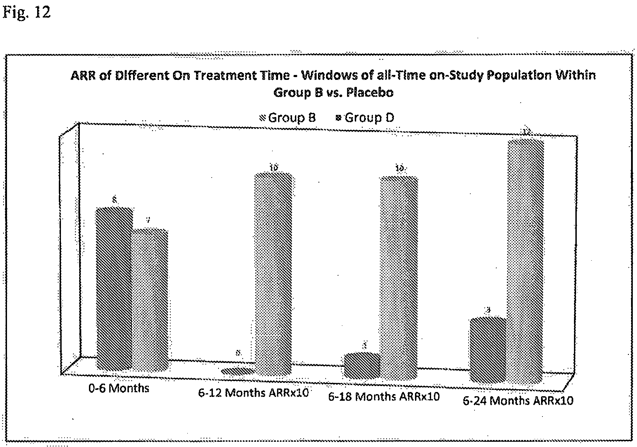

Placebo showed an annual relapse rate of 0.67 during the first six months and increased to 1.00 the second six months period. Then, an annual relapse rate of 1.2, with some minor fluctuations but always with 80-100 percent difference in annual relapse rate compared to B (Table 6, Fig. 10, 12) was reported. Table 9 shows the annual relapse rate within each Group during the 24 months treatment and the percent difference with Placebo. For Group A the annual relapse rate is 0.85 with 18.2 percent decrease compared to placebo (p=0.486, 95% confidence), for Group B 0.40 with 61.5 percent decrease (p=0.014, 95% confidence), for Group C 0.72 with 30.8 percent decrease p=0.175,95% confidence).

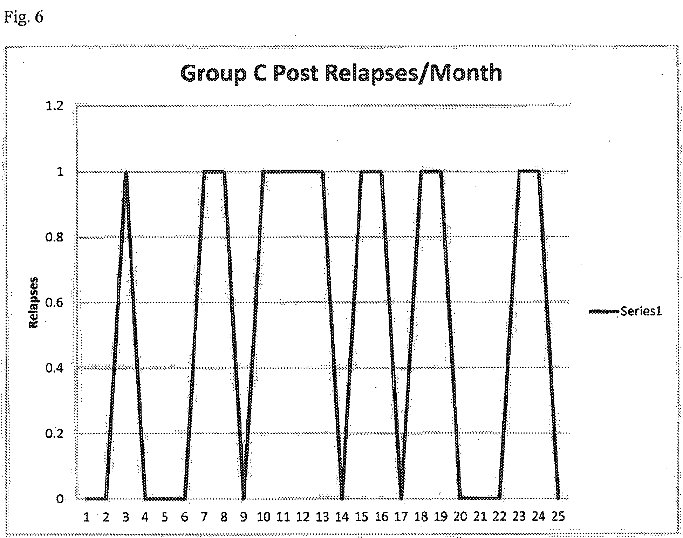

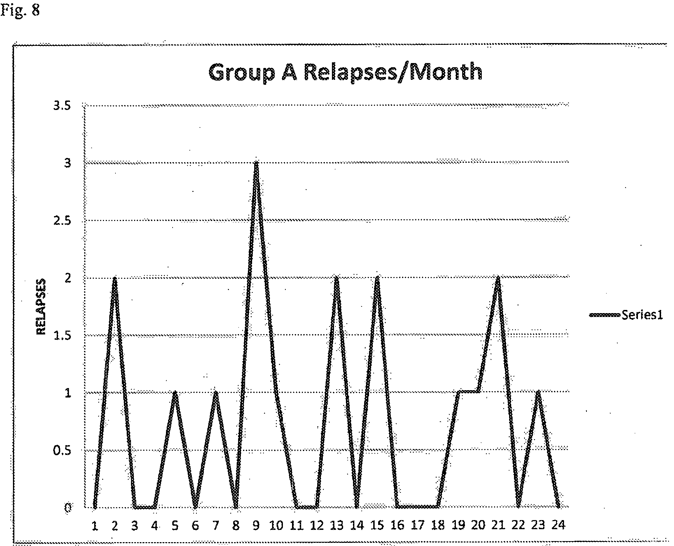

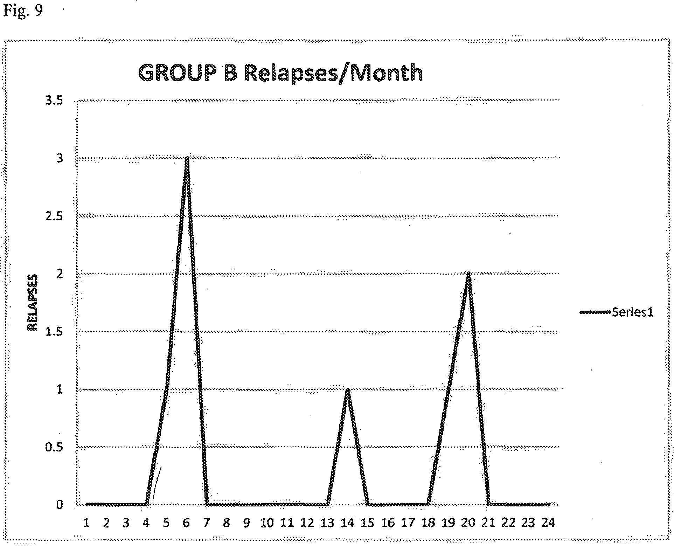

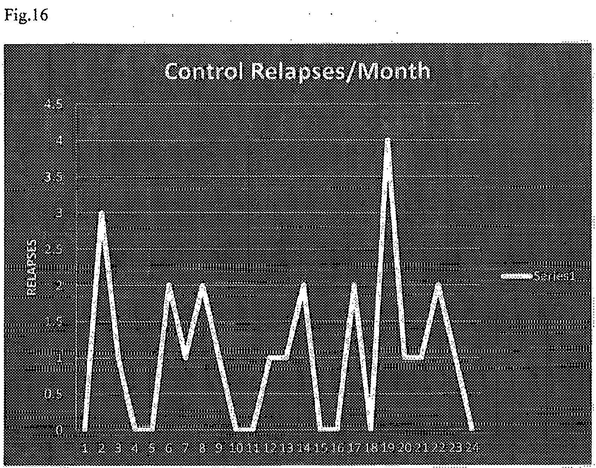

Figures 6, 8, 9 and 16 are the within each group relapses against time where Group B clearly shows an almost regular periodicity/frequency with long relapse free time windows. This phenomenon is important because it is indicative of all drugs that can have a strong positive effect on MS disease since the rule rather than the exception for this disease is the great heterogeneity among patients' disease evolution.

This is unique for Group B since all other groups have an irregular dispersion of relapses with placebo to show the most activity, with relapses dispersion throughout the 2 years period. In Group B all patient showed improvement on relapse frequency. Among them four patients that were considerably active with more than 4 relapses per year before entry resulted with 3 patients with 1 relapse and 1 patient with 2 relapses. An important fact is that the patients in Group B had an annual relapse rate of 1.35 at base line with most patients to have 3 and 4 relapses before entry and patients in placebo 0.83 annual relapse rate, with most patients to have one or two relapses before entry. As discussed above, patients that show accumulation of relapses usually show more relapses activity in contrast with patients with low relapse incidences. For example, for patients with one relapse in two years is not rare to have no attack in the next two years; but it is not common phenomenon with the already existing medicine and disease evolution for some one that had three or more relapses in the past two years to have zero or one relapse in the next two years. After all, the intervention formula B had a strong positive effect on considerably active disease. The above clinical effect had been previously hypothesized during early PUFA studies in MS which stated that the more pronounced effect could be seen in patients with more active disease. This result of most relapse activity within Group B at baseline it was exclusively the result of the drop outs since at entry baseline, of total enrolled population, all groups had about the same mean annual relapse rate.

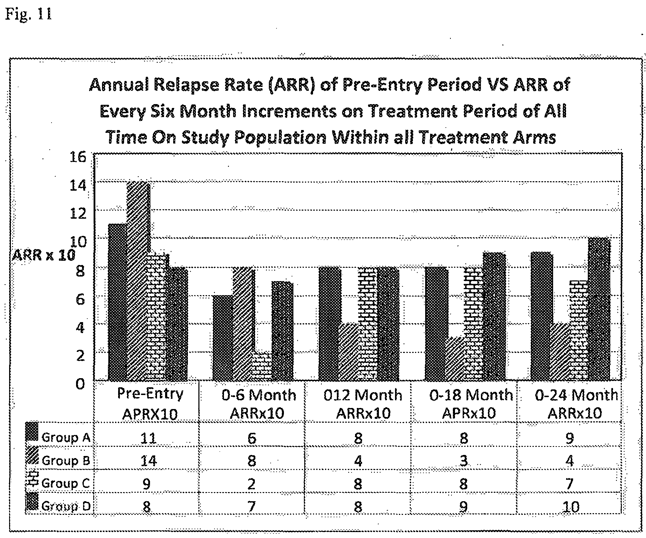

Patients with less activity had the obvious choice to withdraw from the trial since some may have found the taste of the formulation undesirable. On the other hand, we can assume that in the placebo Group more drop outs could be the result of more relapses, meaning no treatment effect within the specific Group. In the placebo Group during treatment period, as we have discussed above, there were more than 60 percent (7 out of 12 patients) of patients that experienced massive relapse incidences meaning equal or more that 3 relapses per patient. Two of the patients within placebo Group switched to more aggressive conventional medication. In Group B only one patient had two relapses and the rest 90 percent were relapse free or with just one relapse. All patients were under normal conventional medication and they followed specific treatment guidelines-protocol as we have discussed above. The number of relapses at every six months period during treatment of all groups is shown in Fig. 10. A comparative ARR of all Groups during pre-entry vs. every six month increments ARR is shown in Fig. 11. The ARR of all-time on-treatment population within different time-windows of Group B as is shown in Fig. 12 where we can clearly observe that for a full year between the 6^{th} month and the 18^{th} month there was only one relapse with annual relapse rate 0.1 within Group B.

Post Study Evaluation (12moths) from Jan 1st 2010 to Dec 31st 2010. All-time on-study patients of all four Groups were followed for additional 12 months after study completion (January 1^{st} 2010 until Dec 31^{st} 2010). All relapse incidences were collected and evaluated. Five relapses were reported for Group A, six relapses for Group B, five relapses for Group C and nineteen relapses for Group D (placebo). During this 12 month extended period the relapse free patients were: 70 percent for Group A, 70 percent for Group B, 55 percent for Group C and only seven percent (93 percent out of the all time on study patients continued relapsing) for placebo. During this period, two patients from Group A, two patients from Group B, one patient from Group C and four patients from the placebo Group D changed to second-line MS drugs (Tysabri®). These results are considered of great interest and additionally confirming the overall efficacy evaluation results of the clinical trial. Additional conclusions arise out of these results such as: a) this might be a result of a long lasting effect by the interventions, b) patients will probably earn much more years of quality life, c) transfer to more aggressive second line drugs might not be needed, d) this might be an additional evidence for probable remyelination and neuroprotection, e) patients may be on a long remission process due to the interventions in contrast to the patients only on DMT treatments, and finally f) the product is novel against all existing treatments that after their discontinuation there is a known rebound effect on relapses and the disease immediately progresses.

### Intention to Treat

Intention to treat is considered the primary analysis for evaluation of the efficacy of the intervention based on all available data obtained. It is a conservative approach that reflects the real clinical practice. Even though our objective is the efficacy of the intervention (proof of concept), we analyze the results according to intention to treat as well. We thoroughly explain the results in order to be clear and understandable.

The phenomenon of large drop out numbers of patients in clinical trials with interventions that contain oils, due to the unpleasant taste and smell, is repeated in our trial as well as in all previous reported oil related trials, even though we had tried to mask the smell and taste with citrus aroma as we have discussed before. By no means has this phenomenon been related to severe adverse or side effects. An analysis of relapses of drop out patients in Group A (n=8), 14 relapses were reported in contrast to 20 before entry (an ARR of 0.88 vs. 1.25 respectively, p=0.306 CI 95%); in Group B (n=7), 14 relapses were reported in contrast to 14 before entry (an ARR of 1.00 vs. 1.00 respectively, p=1.000 CI 95%), in Group C (n=1 0), 26 relapses were reported in contrast to 27 before (an ARR of 1.30 vs. 1.35 respectively, p=0.890 CI 95%) entry and in placebo Group (n=7), 13 attacks were reported in contrast to 20 before entry (an ARR of 0.92 vs. 1.42 respectively, p=0.226 CI 95%) (Table 12). The annual relapse rate of total population (including drop outs) of Group A (n=18), was 1.17 before entry and 0.86 at the end of the study (26.5 percent reduction) with p=0.200, CI 95%; the annual relapse rate of Group B (n=17) was 1.21 before entry and 0.65 at the end of the study (46.3 percent reduction) p=0.019, CI 95%, that is a statistically significant ARR reduction for Group B; the annual relapse rate of Group C (n=19) was 1.13 before entry and 1.03 at the end of the study (8.8 percent reduction) p=0.579, CI 95% and of placebo (n=19), was 1.06 before entry and 1.00 at the end of the study (5.7 percent reduction) p=0.443, CI 95% (Table 13). During the two year study period, in comparison to placebo, Group A presented 14 percent reduction of annual relapse rate (p=0.537 CI 95%), Group B presented a 35 percent reduction of annual relapse rate (p=0.104 CI 95%) where Group C presented a 3 percent increase of the annual relapse rate (p=1 .000 CI 95%).

An overall estimate of the three Groups drop out patients' results is that they do not exhibit any extreme or unexpected outcome. A major part of drop outs, within Placebo Group, needed to start receiving conventional treatment that probably resulted to a decrease of the number of relapses. Within Group A, B and C we had patients with pregnancies. As we have discussed before the patients were uncomfortable about the smell and taste of the syrup formula and they admitted that the main reason for drop out was the palatability of the formulas and not as a result of any other severe adverse event or side effects. As we can see (Table 13) drop out patients when included in the analysis, specifically for Placebo Group, the number of relapses decreases during treatment period compared to pretreatment but also compared to other treatment Groups. A probable reason for this outcome is the known fact that the Placebo Group drop outs most frequently started conventional treatment. Forty three percent (43%) of the Group B drop outs were under conventional treatment at entry base line and remained the same until the end of the study. On the other hand, fifty seven percent (57%) of the Placebo Group drop outs were under conventional treatment at entry base line and this percentage increased to eighty six percent (86%) at the end of the study. Since Interferons and monoclonal antibodies have shown to control in a degree the relapses, we can easily conclude that the effect of these drugs, specifically in Placebo Group drop outs, has affected dramatically the ITT data analysis.

In the ITT analysis of the total population mean disability progression of Group A increased 18.2 percent within the two pre-entry years and 16.2 percent within trial period (10.9 percent reduction); of Group B increased 35.2 percent within the two pre-entry years an 14.9 percent within trial period (57.7 percent reduction); of Group C increased 22.8 percent within the two pre-entry years and 15.3 percent within trial period (32.9 percent reduction) and of Group D increased 19.5 percent within the two pre-entry years and 24.2 percent within trial period (24.1 percent increase) (Table 13). These numbers clearly support the previous statement that the people in trial groups that were in mild stage of the disease (low relapse rate) drop out just because they did not like the taste and the smell or because of pregnancy. Within Group B, 7 drop out patients had only 14 relapses before entry (mild condition) and they are shown to report the same number of attacks during the next two years (within study period). Within Group B two of the patients that dropped out at the very beginning of the study (before successfully completing the normalization period), later became secondary progressive and have been excluded from the analysis of the results (exclusion criteria). One patient was lost to follow-up. As we have previously discussed a significant proportion of the placebo Group drop out patients were put on conventional medication (86% of placebo Group drop outs went on interferon or Tysabri®. Tysabri® has a known decrease on ARR by 68% and decreases the possibility of disability accumulation by 43%. This specific fact positively affects the ITT analysis, in favor of placebo. These parameters and conditions between placebo and treatment intervention if not explain in an. ITT analysis could result to miss-value an otherwise strong treatment efficacy intervention. No pregnancies were reported within placebo Group.

In the ITT analysis mean disability progression of Group A increased 18.2 percent within the two pre-entry years and 16.2 percent within trial period (10.9 percent reduction); of Group B increased 35.2 percent within the two pre-entry years and 14.9 percent within trial period (57.7 percent reduction); of Group C increased 22.8 percent within the two pre-entry years and 15.3 percent within trial period (32.9 percent reduction) and of Group D increased 19.5 percent within the two pre-entry years and 24.2 percent within trial period (24.1 percent increase) (Table 13). These numbers clearly support the previous statements based on the probable reasons for drop outs; limiting those to the palatability of the formula interventions and to the patients' physical condition. More specifically, the mean EDSS score at -24mo pre-entry of ITT patients was 1.59 for Group B, and 2.00 for placebo (Table 13). At entry baseline the mean EDSS score was 2.53 for Group B, and 2.39 for placebo (Table 13). The percentage increase for those pre-entry years until the entry baseline was 35.2 percent for Group B, and 19.5 percent for placebo (Table 13). At the end of the two years treatments study the EDSS for Group B was 2.47, and 2.97 for the placebo. The percentage increase during treatment was 14.9 percent for Group B, and 24.2 percent for placebo (Table 13). Comparing Group B EDSS progression to placebo Group D EDSS progression for the two year period before entry an increased worsening of Group B patients EDSS is observed. When comparing Group B EDSS progression to the placebo Group D EDSS progression during the 2 years of treatment we can still realise a dramatic decrease of the disability progressive course of Group B with intervention formula B. The percentage difference in disability progression of the -24 months (for the two year period before the study) compared to +24 months (for the two years period during study) for Group B is 57.7 percent decrease, and 24.1 percent increase for placebo a significant difference even for ITT analysis.

For the disability progression of total population (ITT) within each one of the groups we can conclude that several factors could positively affected the results mostly as a result of the drop out characteristics (number of patients on conventional treatments and on second-line drug, Tysabri®). All of these parameters as thoroughly previously discussed served a strong scheming role on treatment efficacy result evaluation in favour of the placebo and against the intervention B. Even though all these parameters were in favour of placebo the ITT analysis prove intervention B with strong significant activity against placebo even for ITT.

### EDSS Disability Progression Before and During Treatment of all Time on Study Population

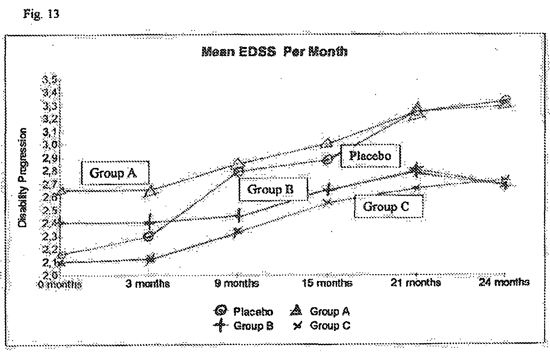

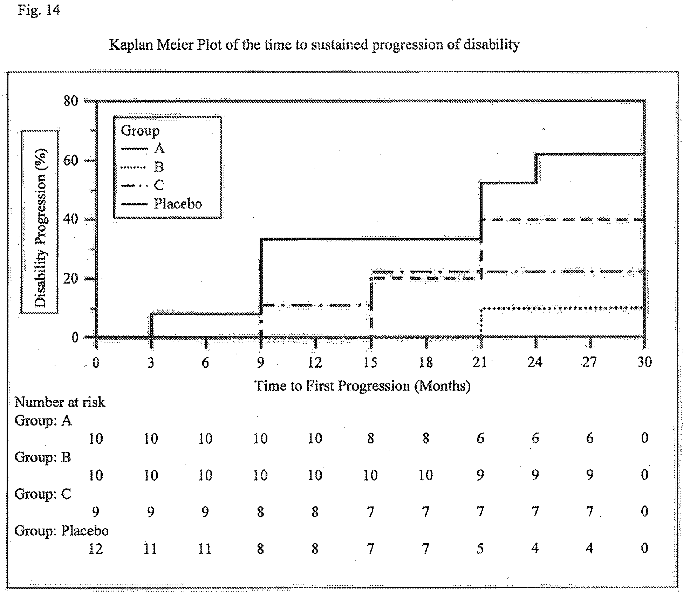

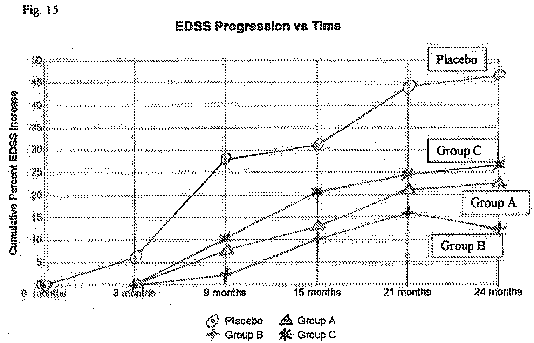

A sustained progression of disability over two years (two-year secondary end point) was significantly less in the intervention formula B Group than in the placebo Group (see Figs. 13 - 15). At two years, the cumulative probability of progression of one point on the EDSS (on the basis of Kaplan Meier analysis) was 10 percent (1/10) in the formula B Group and 58 percent (7/12) in the placebo Group (P=0.049, 95 percent confidence interval, statistically significant), which represents a decrease of 48 percentage points (absolute risk reduction) or a relative (relative risk reduction) 83 percent decrease in the risk of a sustained progression of disability with intervention formula B (Table 11). For Group A, the cumulative probability of progression was 40 percent (4/10) (P=0.301, 95 percent confidence interval), which represents a decrease of 18 percentage points (absolute risk reduction) or a relative (relative risk reduction) 31 percent decrease in the risk of a sustained progression of disability (Table 11). For Group C, the cumulative probability of progression (on the basis of Kaplan Meier analysis) was 22 percent (2/9) (P=0.143, 95 percent confidence interval), which represents a decrease of 36 percentage points (absolute risk reduction) or a relative (relative risk reduction) 62 percent decrease in the risk of a sustained progression of disability (Table 11).

The mean EDSS score at -24mo pre-entry of all time on study patients was 2.05 for Group A, 1.70 for Group B, 2.11 for Group C and 2.08 for placebo (Table 10). At entry baseline the mean EDSS score was 2.65 for Group A, 2.40 for Group B, 2.11 for Group C and 2.16 for placebo (Table 10). The percentage increase for those pre-entry years was 29.3 percent for Group A, 41.2 percent for Group B, 0.0 percent for Group C and 3.8 percent for placebo (Table 10). At the end of the two years study the EDSS for Group A was 3.30, for Group B 2.70, for Group C 2.72 and for the placebo 3.33; the percentage increase during treatment was 24.5 percent for Group A, 12.5 percent for Group B, 28.9 percent for Group C and 54.2 percent for placebo (Table 10). Comparing Group B EDSS progression (41.2 percent increase) to placebo Group EDSS progression (3.8 percent increase) for the two year period before the study we can clearly see the dramatic worsening of Group B patients EDSS. When comparing Group B EDSS progression within the study (12.5 percent increase) to the placebo Group EDSS progression within the study (54.2 percent increase) we can realise a dramatic decrease of the progressive course of Group B with intervention formula B. The disability progression within Group A decreased from 29.3 percent (pre-entry) to 24.5 percent (post-entry) and for Group C increased from 0 percent (pre-entry) to 28.9 percent (post-entry). The percentage difference in disability progression of the -24 months (pre-entry) compare to +24 months (post-entry) for Group A is 16.4 percent decrease, 69.7 percent decrease for Group B, 28.9 percent increase for Group C and 1326.3 percent increase for placebo. Out of ten patients in Group A four patients had an increase of 1 point on EDSS scale and 6 remained stable. Out of ten patients in Group B nine remained stable and one worsened by 1 point on EDSS. Out of nine patients in Group C two patients worsened and seven remained stable and for placebo out of twelve patients seven people worsened and five remained stable. At two years (duration of the clinical trial), the intervention formula B against placebo showed that only 17 percent of patients in Group B had increased risk of worsening disability and about 83 percent of patients remained stable.

### MRI

MRI investigation on T₂-weighted new or enlarging lesions was included as a secondary end point on patients that already had recent MRI scans at the time of enrolment (as a result of their normal medical follow up) in relation to MRI scans of the same patients at the end of the study. The results support the overall conclusion from the study that intervention B has a positive effect on disease activity since only 28 percent from Group B patients, but 67 percent from placebo-Control Group D patients, are shown to have developed new or enlarging T-2 lesions (about forty percentage point deference), 58% relative risk reduction. In addition, the MRI findings show that the development of new or enlarging lesions correlates with the findings of the ARR and disability accumulation differences.

### Safety

Over the course of the 30 month study no significant adverse events were reported from any group. According to a questioner procedure the only aetiology for drop-outs was the palatability and smell of the formula preparations. Nausea was reported by two patients. No abnormal values observed on any of the biochemical and haematological blood tests. No allergic reactions reported.

### Statistical Analysis

According to small size clinical trial statistical analysis guidelines, more than one statistical method has to be applied in order to confirm the validity of the result. Here, three different statistical methods are applied for the analysis of relapses, Poisson, Quassi Poisson regression and percent difference, and three different statistical methods for the analysis of EDSS scores, the proportion progressing (*Kaplan Meier*), within mean population change EDSS (Wilcoxon rank test) and the sophisticated Series method that is lately suggested by a group of Harvard Researchers and refer to the work of our statistician (Micha Mandel et al. 2007). More specifically logistic regression model by using likelihood methods and employing the Gauss---Hermite quadrature methods is employed. The percent difference was also performed for EDSS progression. All methods used give approximately the same outcome, statistically significant efficacy (p<0.05, CI 95%) of Intervention B with ∼80%, α=0.05 statistical power (post-hoc).

### Discussion

Multiple sclerosis (MS) is an inflammatory demyelinating disease of the central nervous system (CNS) that destroys myelin, oligodendrocytes (myelin-forming cells of the CNS), and axons with an unknown etiology. Once established, the disease is considered to be immune-mediated where the immune cells attack the myelin sheaths of neurons. T cells and macrophages are thought to be involved in demyelination through various mechanisms. B cells have direct effects on immune regulation and brain destruction. B-cells secrete Interleukin-6 (IL-6), Interleukin-10 (IL-10), tumor necrosis factor (TNF-a) and chemokines. They also express high levels of costimulatory molecules (CD80) in patients with relapsing MS. As a result, they are potent antigen presenting cells (APC) because they are exquisitely focused against specific antigens. New insights suggest oligodendrocyte apoptosis to be a primary event accompanied by microglial activation. Subsequently, T cells and macrophages become activated and migrate into the lesion area. The important pathological mechanisms involved in MS include immune mediated inflammation, oxidative stress and excitotoxicity. These mechanisms may all contribute to oligodendrocyte and neuronal damage and even cell death, hence promoting disease progression.

Intervention formula B (with the acronym "PLP 10") is unlike any formulation of the prior art in that it contains EPA, DHA, LA, GLA, other omega-3 PUFA, MUFA, SFA, Vitamin A, Vitamin E and γ-tocopherol, and resulted in statistically significant improvements in treatment to a much greater degree than prior treatments. It reduced the probability of a patient's disability to worsen by one point on the EDSS by 83% in comparison to placebo. This is a significant advance over conventional therapies such as DMT, which decreased the probability by 18%.

In patients with relapsing multiple sclerosis, intervention formula B significantly reduced the risk of progression of disability and the annualized relapse rate over two years of treatment. The positive effect of the Intervention formula B is greater than any mild conventional medical therapy and the same or even better than the second line more toxic existing therapies but free of their severe side effects. The effect of intervention formula B was recorded after six months of treatment and was sustained throughout the study. Disease-modifying therapies have become the cornerstone of treatment for patients with relapsing multiple sclerosis for the last 20 years. The two-year trials of the therapies that are currently available (interferon beta products and glatiramer acetate) have shown that these agents reduce the annualized relapse rate by about one third (PRISMS Study Group. 1998, OWIMS 1999, Yong VW, et al. 1998, Beck RW, et al. 1992). In addition, Phase IV, post-marketing studies have shown that the 30 percent annualized relapse rate reduction remains for about 10 to 25 years, until today, with no major impact on

EDSS progression. Hence, there remains a need for more effective treatments for relapsing multiple sclerosis.

This specific intervention B sustains years of quality of life, particularly when it is used from the early stages of the disease. Our study provides strong evidence that intervention formula B in patients with relapsing multiple sclerosis significantly reduces:
(a) the disease risk probability of disability progression by one point on EDSS by 83 percent compared to the placebo-control (83 percent remained stable in relation to the placebo);
(b) the development of clinical relapses in patients with relapsing multiple sclerosis (about 61.5 percent against the placebo and more than 70.4 percent decrease compared to the two pre-entry years annual relapse rate); and
(c) the appearance of new or enlarging T-2 lesions (about 40 percentage points difference with placebo by brain MRI). Based on previous observations and on our results, we believe that the effect of intervention formula B on early stages of the disease is a major advance in the treatment of MS.
Due to its proven strong efficacy, the nature of the formulation and no associated side effects, intervention formula B can be used as a preventive treatment during the prodromal phase of the disease, another major advance in the treatment of complex neurodegenerative diseases and MS.

Intervention formula B could result in improved remyelination and neuroprotection, thereby contributing to the improved EDSS score in our trial. Moreover, our data indicate that efficacy is observed early after supplementation and persists throughout the treatment period. Within the 30-month evaluation period of this trial (including normalization period), intervention formula B had an excellent safety outcome without any reported severe adverse events. Safety is a primary important characteristic required out of a treatment, it is for sure a proved fact that our formula is the only one without any side effects among everything else

From the outcome of this clinical study is more than conclusive that annual relapse rate is significantly decreased with this specific intervention B formula. Continued assessments of long-term treatment with intervention formula B will better establish its place in the arsenal of treatments for relapsing multiple sclerosis. Formula B (and formulations like it described throughout this application) appears to be the best choice treatment out of the limited existing treatment agents for MS.

This clinical trial results are of high value since no other similar investigation exists or ever published providing strong link evidence between, dietary, metabolic, immunological and neurobiological aspects of MS; therefore for the first time we can begin to make a sense of the wealth benefits of apparently unconnected aspects of MS, particularly in relation to dietary fats. Our Formula at the end of 2-year study reduces relapses by 61.5% in comparison with the placebo.

Our Formula reduces the probability of a patient's disability to worsen by one point on the EDSS by 83% in comparison with the placebo. Our formula is also differentiated from the prior art because it surprisingly indicates that its efficacy is also characterized by long free relapse period compared to placebo (periodicity and regular frequency).

There is a long lasting effect and almost has the same or even better efficacy when used as an adjuvant, as the second-line drugs for MS. This is proved by the 12 month extension of collecting data (post study). There is a strong possibility of remyelination and neuroprotection. An ITT analysis supports the results. The evaluations of the trial are out of more than a total of 5 years (2 years pre-study evaluation + 2 years on study + 1 year post study evaluation) follow-up of the patients in relation to the trial that gives this study dynamics and power on the result evaluation and conclusions.

Intervention B increases the probability of having one or less than one relapse over two year period by 114 percent compared to placebo. (See Fig. 11, 12). The sustained progression of disability over two years was significantly less in the intervention formula B group than in the Placebo group.

There is 83 percent relative risk reduction of a sustained progression of disability compared to Placebo. That means, only 17 percent of patients on Intervention B treatment had risk of worsening disability and about 83 percent of patients remained stable against placebo. These results therefore confirm and demonstrate unequivocally that the specific formulation regime has a strong therapeutic effect with no side effects, better than anything before this.

The present inventors have now found a preparation for the treatment of MS that is effective because it provides simultaneous and effective activity on the function of the total pathophysiological pathways involved and neurodegenerative mechanisms, and at the same time orchestrates the activation of the restoration and neuroprotection pathways, which is important for influencing the etiology and development of a wide range of neurodegenerative diseases and autoimmune diseases/disorders. The present invention is a preparation for the treatment of MS that considers for the first time the complex multifactorial nature of the disease and the interconnected network of events and factors, according to the systems medicine concept through systems biology and nutritional systems biology approach model, for new avenues of safer and more effective treatment of complex multifactorial diseases and MS.

Moreover, intervention B, may be effective in treating other types of MS (primary progressive, secondary progressive, progressive relapsing).

The use of the terms "a" and "an" and "the" in the context of this disclosure (especially in the context of the following claims) are to be construed to cover both the singular and the plural, unless otherwise indicated herein or clearly contradicted by context.

Alternative embodiments of the claimed invention are described herein, including the best mode known to the inventors for carrying out the claimed invention. The disclosure of ranges is intended as a continuous range including every value between the minimum and maximum values The recitation of ranges of values herein are merely intended to serve as a shorthand method of referring individually to each separate value falling within the range, unless otherwise indicated herein, and each separate value is incorporated into the specification as if it there individually recited herein.

It is to be understood that any ranges, ratios and ranges of ratios that can be formed by, or derived from, any of the data disclosed herein represent further embodiments of the present disclosure and are included as part of the disclosure as though they were explicitly set forth. This includes ranges that can be formed that do or do not include a finite upper and/or lower boundary. Accordingly, a person of ordinary skill in the art most closely related to a particular range, ratio or range of ratios will appreciate that such values are unambiguously derivable from the data presented herein.

## Claims

1. A liquid oral composition selected from the group consisting of a pharmaceutical, nutritional, medical food, functional food, clinical nutrition, medical nutrition or dietetic preparation, comprising
a long chain polyunsaturated fatty acid (PUFA) fraction, comprising eicosapentaenoic acid (EPA), docosahexaenoic acid (DHA), linoleic acid (LA) and gamma linolenic acid (GLA); one or more other omega-3 PUFAs; and
one or more monounsaturated fatty acid (MUFA);
and wherein the EPA is present in an amount of 500 mg to 5000 mg;
and/or wherein the DHA is present in an amount of 1000 mg to 12000 mg.

2. The composition according to claim 1 further comprising a saturated fatty acid (SFA).

3. The composition according to claim 1 further comprising a vitamin selected from the group consisting of Vitamin A, Vitamin E and gamma-tocopherol.

4. The composition according to claim 1 wherein the LA is present in an amount of 1000 mg to 10600 mg.

5. The composition according to claim 1 wherein the GLA is present in an amount of 1000 mg to 16000 mg.

6. The composition according to claim 1 further comprising gamma-tocopherol.

7. The composition according to claim 6 wherein the gamma-tocopherol is present in an amount of 100 mg to 3000 mg.

8. The composition according to claim 1 further comprising beta-carotene.

9. The composition according to claim 8 wherein the beta-carotene is present in an amount of 0.1 mg to 5 mg.

10. The composition according to claim 1 wherein the monounsaturated fatty acid is selected from the group consisting of 18:1 (oleic acid), 20:1 (eicosenoic acid), 22:1 (docosenoic acid), 24: 1 (tetracosenic acid), and mixtures of the foregoing.

11. The composition according to claim 2 wherein the SFA is selected from the group consisting of 16:0 (palmitic acid) and 18:0 (stearic acid), and mixtures of the foregoing.

12. The composition according to claim 1 wherein the other omega-3 PUFA is selected from the group consisting of 18:3 (alpha-linolenic acid), 18:4 (stearidonic acid), 20:4 (eicosatetraenoic acid), and 22:5 (docosapentaenoic acid), and mixture of the foregoing.

13. The composition of claim 12 wherein the other omega-3 PUFA is present in an amount of 100 mg to 2500 mg.

14. The composition of claim 13 wherein the other omega-3 PUFA is present in an amount of 300 mg to 2000 mg.

15. The composition of claim 13 wherein the other omega-3 PUFA is present, in an amount of 600 mg to 1000 mg.

16. The composition of claim 1 wherein the MUFA is present in an amount of 100 mg to 3500 mg.

17. The composition of claim 1 wherein the MUFA is present in an amount of 750 mg to 3500 mg.

18. The composition of claim 1 wherein the MUFA is present in an amount of 1500 mg to 3500 mg,

19. The composition of claim 11 wherein the SFA is present in an amount of 500 mg to 2000 mg.

20. A liquid oral composition according to any one of the preceding claims 1 to 19, comprising:
(a) 1650 mg EPA;
(b) 4650 mg DHA;
(c) 3850 mg LA;
(d) 5850 mg GLA;
(e) 760 mg gamma-tocopherol, and
(f) 22 mg Vitamin E.

21. A liquid oral composition according to any one of the preceding claims 1 to 19, comprising:
a. EPA 1650 mg/dose
b. DHA 4650 mg/dose
c. GLA 2000 mg/dose
d. LA 3850 mg/dose
e. Other omega-3 PUFAs 600 mg/dose, comprising:
i. Alpha-linolenic acid (C18:3n-3) 37 mg/dose
ii. Stearidonic acid (C18:4n-3) 73 mg/dose
iii. Eicosatetraenoic acid (C20:4n-3) 98 mg/dose
iv. Docosapentaeooic acid (C22:5n-3) 392 mg/dose
f. MUFAs, comprising:
i. 18:1 - 1300 mg/dose
ii. 20:1 - 250 mg/dose
iii. 22:1 - 82 mg/dose
iv. 24:1 - 82 mg/dose
g. SFAs, comprising:
i. 18:1 - 160 mg dose
ii. 16:0 - 650 mg/dose
h. Vitamin A 0.6 mg/dose
i. Vitamin E 22 mg/dose
j. Gamma-tocopherol 760 mg/dose

22. A liquid composition according to claim 1 for use in treating or preventing a neurodegenerative disease in a human subject comprising:
(a) 500 mg to 5000 mg EPA;
(b) 1000 mg to 12000 mg DHA;
(c) 1000 mg to 10600 mg LA; and
(d) 1000 mg to 16000 mg GLA.

23. The composition for use according to claim 22 wherein the composition is for administering once daily.

24. The composition for use according to claim 22 wherein the composition is for administering once daily for more than 30 days.

25. The composition for use according to claim 22 wherein the disease is multiple sclerosis.

26. The composition for use according to claim 22 wherein the composition comprises:
(a) 1,650 mg EPA;
(b) 4650 mg DHA;
(c) 3850 mg LA;
(d) 5850 mg GLA;
(e) 760 mg gamma-tocopherol;
(f) 22 mg Vitamin E; and
(g) 0.6 mg beta-carotene.

27. The composition for use according to claim 22 wherein the composition is for administering for more than 60 consecutive days.

28. The composition according to any one of the claims 1 to 27 for use in the prevention and/or treatment of neurodegenerative and/or autoimmune diseases and syndromes, especially of MS, and/or for spinal cord injury recovery; and/or for use in the prevention and/or treatment of psychiatric disease, degenerative disease, autoimmune disease, immune mediated inflammation, inflammation, cardiovascular disease, epileptogenesis and/or epilepsy.

29. The composition according to claim 28 for use in the prevention and/or treatment of neurodegenerative and/or autoimmune diseases and syndromes, especially of MS, and/or for use in spinal cord injury recovery; preferably for use in the prevention and/or treatment of MS.

30. The composition according to any one of the claims 1 to 27 for use in treating or preventing diseases and/or syndromes selected from the group consisting of neurological, (neuro)degenerative, psychological and autoimmune diseases and/or disorders, in particular Huntington's disease, Parkinson's disease, Alzheimer's disease and/or dementias; and/or for use as an adjuvant to conventional drugs for treating and/or preventing these diseases or syndromes.

## Patentansprüche

1. Eine flüssige orale Zusammensetzung, ausgewählt aus der Gruppe bestehend aus einem Pharmazeutikum, Ernährungsmittel, medizinisches Nahrungsmittel, funktionelles Nahrungsmittel, klinische Ernährung, medizinische Ernährung oder diätetische Zubereitung, umfassend
eine langkettige mehrfach ungesättigte Fettsäure (PUFA)-Fraktion, enthaltend Eicosapentaensäure (EPA), Docosahexaensäure (DHA), Linolsäure (LA) und Gamma-Linolensäure (GLA); eine oder mehrere andere Omega-3-PUFAs; und eine oder mehrere einfach ungesättigte Fettsäure (MUFA);
und wobei die EPA in einer Menge von 500 mg bis 5000 mg vorliegt;
und/oder wobei die DHA in einer Menge von 1000 mg bis 12000 mg vorliegt.

2. Zusammensetzung nach Anspruch 1, weiterhin enthaltend eine gesättigte Fettsäure (SFA).

3. Zusammensetzung nach Anspruch 1, weiterhin enthaltend ein Vitamin, ausgewählt aus der Gruppe bestehend aus Vitamin A, Vitamin E und Gamma-Tocopherol.

4. Zusammensetzung nach Anspruch 1, wobei die LA in einer Menge von 1000 mg bis 10600 mg vorliegt.

5. Zusammensetzung nach Anspruch 1, wobei die GLA in einer Menge von 1000 mg bis 16000 mg vorliegt.

6. Zusammensetzung nach Anspruch 1, weiterhin enthaltend Gamma-Tocopherol.

7. Zusammensetzung nach Anspruch 6, wobei das Gamma-Tocopherol in einer Menge von 100 mg bis 3000 mg vorliegt.

8. Zusammensetzung nach Anspruch 1, weiterhin enthaltend Beta-Karotin.

9. Zusammensetzung nach Anspruch 8, wobei das Beta-Karotin in einer Menge von 0,1 mg bis 5 mg vorliegt.

10. Zusammensetzung nach Anspruch 1, wobei die einfach ungesättigte Fettsäure ausgewählt ist aus der Gruppe bestehend aus 18:1 (Ölsäure), 20:1 (Eicosensäure), 22:1 (Docosensäure), 24:1 (Tetracosensäure) und Mischungen der vorangehenden.

11. Zusammensetzung nach Anspruch 2, wobei die SFA ausgewählt ist aus der Gruppe bestehend aus 16:0 (Palmitinsäure) und 18:0 (Stearinsäure) und Mischungen der vorangehenden.

12. Zusammensetzung nach Anspruch 1, wobei die andere Omega-3-PUFA ausgewählt ist aus der Gruppe bestehend aus 18:3 (Alpha-Linolensäure), 18:4 (Stearidonsäure) 20:4 (Eicosatetraensäure) und 22:5 (Docosapentaensäure) und Mischung der vorangehenden.

13. Zusammensetzung nach Anspruch 12, wobei die andere Omega-3-PUFA in einer Menge von 100 mg bis 2500 mg vorliegt.

14. Zusammensetzung nach Anspruch 13, wobei die andere Omega-3-PUFA in einer Menge von 300 mg bis 2000 mg vorliegt.

15. Zusammensetzung nach Anspruch 13, wobei die andere Omega-3-PUFA in einer Menge von 600 mg bis 1000 mg vorliegt.

16. Zusammensetzung nach Anspruch 1, wobei die MUFA in einer Menge von 100 mg bis 3500 mg vorliegt.

17. Zusammensetzung nach Anspruch 1, wobei die MUFA in einer Menge von 750 mg bis 3500 mg vorliegt.

18. Zusammensetzung nach Anspruch 1, wobei die MUFA in einer Menge von 1500 mg bis 3500 mg vorliegt.

19. Zusammensetzung nach Anspruch 11, wobei die SFA in einer Menge von 500 mg bis 2000 mg vorliegt.

20. Flüssige orale Zusammensetzung nach einem der vorhergehenden Ansprüche 1 bis 19, umfassend:
(a) 1650 mg EPA;
(b) 4650 mg DHA;
(c) 3850 mg LA;
(d) 5850 mg GLA;
(e) 760 mg Gamma-Tocopherol, und
(f) 22 mg Vitamin E.

21. Flüssige orale Zusammensetzung nach einem der vorhergehenden Ansprüche 1 bis 19, umfassend:
a. EPA 1650 mg/Dosis
b. DHA 4650 mg/Dosis
c. GLA 2000 mg/Dosis
d. LA 3850 mg/Dosis
e. Andere Omega-3-PUFAs 600 mg/Dosis, enthaltend:
i. Alpha-Linolensäure (C18:3n-3) 37 mg/Dosis
ii. Stearidonsäure (C18:4n-3) 73 mg/Dosis
iii. Eicosatetraensäure (C20:4n-3) 98 mg/Dosis
iv. Docosapentaensäure (C22:5n-3) 392 mg/Dosis
f. MUFAs, enthaltend:
i. 18:1 - 1300 mg/Dosis
ii. 20:1 - 250 mg/Dosis
iii. 22:1 - 82 mg/Dosis
iv. 24:1 - 82 mg/Dosis
g. SFAs, enthaltend:
i. 18:1 - 160 mg/Dosis
ii. 16:0 - 650 mg/Dosis
h. Vitamin A 0,6 mg/Dosis
i. Vitamin E 22 mg/Dosis
j. Gamma-Tocopherol 760 mg/Dosis

22. Flüssige Zusammensetzung nach Anspruch 1 zur Verwendung bei der Behandlung oder Prävention einer neurodegenerativen Erkrankung in einem menschlichen Subjekt, enthaltend:
(a) 500 mg bis 5000 mg EPA;
(b) 1000 mg bis 12000 mg DHA;
(c) 1000 mg bis 10600 mg LA; und
(d) 1000 mg bis 16000 mg GLA.

23. Zusammensetzung zur Verwendung nach Anspruch 22, wobei die Zusammensetzung für die einmal tägliche Verabreichung vorgesehen ist.

24. Zusammensetzung zur Verwendung nach Anspruch 22, wobei die Zusammensetzung für die einmal tägliche Verabreichung über mehr als 30 Tage vorgesehen ist.

25. Zusammensetzung zur Verwendung nach Anspruch 22, wobei die Erkrankung Multiple Sklerose ist.

26. Zusammensetzung zur Verwendung nach Anspruch 22, wobei die Zusammensetzung enthält:
(a) 1650 mg EPA;
(b) 4650 mg DHA;
(c) 3850 mg LA;
(d) 5850 mg GLA;
(e) 760 mg Gamma-Tocopherol;
(f) 22 mg Vitamin E; und
(g) 0,6 mg Beta-Karotin.

27. Zusammensetzung zur Verwendung nach Anspruch 22, wobei die Zusammensetzung für die Verabreichung über mehr als 60 aufeinanderfolgende Tage vorgesehen ist.

28. Zusammensetzung nach einem der Ansprüche 1 bis 27 zur Verwendung bei der Prävention und/oder Behandlung von neurodegenerativen und/oder Autoimmun-Erkrankungen und Syndromen, insbesondere von MS, und/oder zur Wiedergesundung von Rückenmarksverletzung; und/oder zur Verwendung bei der Prävention und/oder Behandlung von psychiatrischer Erkrankung, degenerativer Erkrankung, Autoimmun-Erkrankung, immunvermittelter Entzündung, Entzündung, kardiovaskulärer Erkrankung, Epileptogenese und/oder Epilepsie.

29. Zusammensetzung nach Anspruch 28 zur Verwendung bei der Prävention und/oder Behandlung von neurodegenerativen und/oder Autoimmun-Erkrankungen und Syndromen, insbesondere von MS, und/oder zur Verwendung zur Wiedergesundung von Rückenmarksverletzung; vorzugsweise zur Verwendung bei der Prävention und/oder Behandlung von MS.

30. Zusammensetzung nach einem der Ansprüche 1 bis 27 zur Verwendung bei der Behandlung oder Prävention von Erkrankungen und/oder Syndromen, ausgewählt aus der Gruppe bestehend aus neurologischen, (neuro)degenerativen, psychologischen und Autoimmun-Erkrankungen und/oder Beschwerden, insbesondere Huntington'sche Erkrankung, Parkinson'sche Erkrankung, Alzheimer'sche Erkrankung und/oder Demenzen; und/oder zur Verwendung als ein Adjuvans für herkömmliche Arzneimittel zur Behandlung und/oder Prävention dieser Erkrankungen oder Syndrome.

## Revendications

1. Composition orale liquide choisi dans le groupe être constitué par un produit pharmaceutique, produit nutritionnel, aliment médical, aliment fonctionnel, nutrition clinique, nutrition médicale ou préparation diététique, comprenant
une fraction de longue chaîne d'acide gras polyinsaturé (AGPI) comprenant de l'acide eicosapentaénoïque (EPA), l'acide docosahexaénoïque (DHA), l'acide linoléique (AL) et l'acide gamma-linolénique (AGL); un ou plusieurs autres oméga-3-AGPIs; et
un ou plusieurs acide gras mono-insaturé (AGMI);
et dans laquelle l'EPA est présent en une quantité de 500 mg à 5000 mg;
et/ou dans laquelle le DHA est présent en une quantité de 1000 mg à 12000 mg.

2. Composition selon la revendication 1, comprenant en outre un acide gras saturé (AGS).

3. Composition selon la revendication 1, comprenant en outre une vitamine choisie dans le groupe constitué par vitamine A, vitamine E et gamma-tocophérol.

4. Composition selon la revendication 1, dans laquelle l'AL est présent en une quantité de 1000 mg à 10600 mg.

5. Composition selon la revendication 1, dans laquelle l'AGL est présent en une quantité de 1000 mg à 16000 mg.

6. Composition selon la revendication 1, comprenant en outre gamma-tocophérol.

7. Composition selon la revendication 6, dans laquelle le gamma-tocophérol est présent en une quantité de 100 mg à 3000 mg.

8. Composition selon la revendication 1, comprenant en outre du bêta-carotène.

9. Composition selon la revendication 8, dans lequel le bêta-carotène est présent en une quantité de 0,1 mg à 5 mg.

10. Composition selon la revendication 1, dans laquelle l'acide gras mono-insaturé est choisi dans le groupe constitué par le 18:1 (acide oléique), 20:1 (acide éicosénoïque), 22:1 (acide docosénoïque), 24:1 (acide tetracosénoïque) et des mélanges des précédents.

11. Composition selon la revendication 2, dans laquelle le AGS est choisi dans le groupe constitué par le 16:0 (acide palmitique) et 18:0 (acide stéarique) et des mélanges des précédents.

12. Composition selon la revendication 1, dans laquelle l'autre AGPI oméga-3 est choisi dans le groupe constitué par le 18:3 (acide alpha-linolénique), 18:4 (acide stéaridonique) 20:4 (acide eicosatétraénoïque) et 22:5 (acide docosapentaénoïque) et des mélanges des précédents.

13. Composition selon la revendication 12, dans laquelle l'autre AGPI oméga-3 est présent en une quantité de 100 mg à 2500 mg.

14. Composition selon la revendication 13, dans laquelle l'autre AGPI oméga-3 est présent en une quantité de 300 mg à 2000 mg.

15. Composition selon la revendication 13, dans laquelle l'autre AGPI oméga-3 est présent en une quantité de 600 mg à 1000 mg.

16. Composition selon la revendication 1, dans laquelle l'AGMI est présent en une quantité de 100 mg à 3500 mg.

17. Composition selon la revendication 1, dans laquelle l'AGMI est présent en une quantité de 750 mg à 3500 mg.

18. Composition selon la revendication 1, dans laquelle l'AGMI est présent en une quantité de 1500 mg à 3500 mg.

19. Composition selon la revendication 11, dans laquelle l'AGS est présent en une quantité de 500 mg à 2000 mg.

20. Composition orale liquide selon l'une quelconque des revendications précédentes 1 à 19, comprenant:
(a) 1650 mg EPA;
(b) 4650 mg DHA;
(c) 3850 mg AL;
(d) 5850 mg AGL;
(e) 760 mg gamma-tocophérol, et
(f) 22 mg vitamine E.

21. Composition orale liquide selon l'une quelconque des revendications précédentes 1 à 19, comprenant:
a. EPA 1650 mg/dose
b. DHA 4650 mg/dose
c. AGL 2000 mg/dose
d. AL 3850 mg/dose
e. Autres AGPI oméga-3 600 mg/dose, comprenant:
i. L'acide alpha-linolénique (C18:3n-3) 37 mg/dose
ii. L'acide stéaridonique (C18:4n-3) 73 mg/dose
iii. L'acide eicosatétraénoïque (C20:4n-3) 98 mg/dose
iv. L'acide docosapentaénoïque (C22:5n-3) 392 mg/dose
f. AGMIs, comprenant:
i. 18:1 - 1300 mg/dose
ii. 20:1 - 250 mg/dose
iii. 22:1 - 82 mg/dose
iv. 24:1 - 82 mg/dose
g. AGSs, comprenant:
i. 18:1 - 160 mg/dose
ii. 16:0 - 650 mg/dose
h. Vitamine A 0,6 mg/dose
i. Vitamine E 22 mg/dose
j. Gamma-tocophérol 760 mg/dose

22. Composition liquide selon la revendication 1 pour l'utilisation dans le traitement ou la prévention d'une maladie neurodégénérative dans un sujet humain comprenant:
(a) 500 mg à 5000 mg EPA;
(b) 1000 mg à 12000 mg DHA;
(c) 1000 mg à 10600 mg AL; et
(d) 1000 mg à 16000 mg AGL.

23. Composition pour l'utilisation selon la revendication 22, dans laquelle la composition est destinée à l'administration une fois par jour.

24. Composition pour l'utilisation selon la revendication 22, dans laquelle la composition est destinée à l'administration une fois par jour pendant plus de 30 jours.

25. Composition pour l'utilisation selon la revendication 22, dans laquelle la maladie est la sclérose en plaques.

26. Composition pour l'utilisation selon la revendication 22, dans laquelle la composition comprend:
(a) 1650 mg EPA;
(b) 4650 mg DHA;
(c) 3850 mg AL;
(d) 5850 mg AGL;
(e) 760 mg gamma-tocophérol;
(f) 22 mg Vitamine E; et
(g) 0,6 mg bêta-carotène.

27. Composition pour l'utilisation selon la revendication 22, dans laquelle la composition est destinée à l'administration de plus de 60 jours consécutifs.

28. Composition selon l'une quelconque des revendications 1 à 27 pour l'utilisation dans la prévention et/ou le traitement de maladies et syndromes neurodégénératives et/ou auto-immunes, en particulier de la SEP (sclérose en plaques), et/ou pour la rétablissement de lésion de la moelle épinière; et/ou pour l'utilisation dans la prévention et/ou traitement de maladie psychiatrique, maladie dégénérative, maladie auto-immune, inflammation à médiation immunitaire, inflammation, maladie cardiovasculaire, épileptogenèse et/ou épilepsie.

29. Composition selon la revendication 28 pour l'utilisation dans la prévention et/ou le traitement de maladies et syndromes neurodégénératives et/ou auto-immunes, en particulier de la SEP, et/ou pour l'utilisation dans la rétablissement de lésion de la moelle épinière; de préférence pour l'utilisation dans la prévention et/ou le traitement de la SEP.

30. Composition selon l'une quelconque des revendications 1 à 27 pour l'utilisation dans le traitement ou la prévention de maladies et/ou syndromes choisi dans le groupe constitué par de maladies et/ou troubles neurologiques, (neuro)dégénératives, psychologiques et auto-immunes, en particulier maladie de Huntington, maladie de Parkinson, maladie d'Alzheimer et/ou démences; et/ou pour l'utilisation comme un adjuvant à des médicaments conventionnels pour le traitement et/ou la prévention de ces maladies ou syndromes.
